# EUROPÄISCHE PATENTSCHRIFT

(11) **EP 3 305 232 B1**
(45) Veröffentlichungstag und Bekanntmachung des Hinweises auf die Patenterteilung: **25.08.2021**
(21) Anmeldenummer: 18152034.7
(22) Anmeldetag: 17.01.2018
(51) Int. Cl.: A61B 34/00, B25J 13/06, B25J 19/06, G06F 3/01, G02B 27/01, A61B 90/00

(54) **BEDIENVORRICHTUNG UND BEDIENVERFAHREN ZUM BEDIENEN EINES MEDIZINISCHEN GERÄTS**
CONTROL DEVICE AND CONTROL METHOD FOR OPERATING A MEDICAL DEVICE
DISPOSITIF DE COMMANDE ET PROCEDE DE COMMANDE POUR L'OPÉRATION D'UN DISPOSITIF MÉDICAL

(30) Priorität: 16.02.2017 DE 102017202517
(43) Veröffentlichungstag der Anmeldung: 11.04.2018
(73) Patentinhaber: Siemens Healthcare GmbH, 91052 Erlangen (DE)
(72) Erfinder: Schweizer, Hans, 94447 Plattling (DE)

(56) Entgegenhaltungen:
- DE-A1-102006 019 864
- DE-A1-102013 104 429
- DE-A1-102014 217 559
- DE-A1-102015 204 767
- US-A1- 2014 002 337

## Beschreibung

Die Erfindung betrifft eine Bedienvorrichtung für ein medizinisches Gerät mit einer Projektionseinrichtung, welche zur reellen oder virtuellen Projektion einer Bedienoberfläche auf ein Darstellungselement eingerichtet ist. Die Erfindung betrifft zudem ein Bedienverfahren zum Bedienen eines medizinischen Gerätes mittels einer derartigen Bedienvorrichtung.

Die DE 10 2015 204 767 A1 befasst sich mit der Bedienung eines Medizingeräts. Dazu hat eine Bedieneinheit eine Projektionseinheit zum Projizieren einer Bedienoberfläche auf eine Projektionsfläche. Mit einer Aufnahmeeinheit wird eine Bedienaktion in Form einer Bewegung in einem Projektionsvolumen aufgenommen, welche dann in ein Bediensignal umgewandelt wird. Das Medizingerät und/oder die Projektionseinheit wird dann mit Hilfe eines auf der Grundlage des Bediensignals erzeugten Steuersignals angesteuert.

Die DE 10 2014 217 559 A1 offenbart ein Benutzerassistenzsystem einer Reinigungs- und Desinfektionseinrichtung. Das System umfasst eine Datenbrille, eine Kamera und ein bildgebendes optisches Modul zum Projizieren von Informationen auf eine im Blickfeld eines Benutzers der Datenbrille angeordnete Projektionsfläche. Mit der Kamera wird ein Bild eines chirurgischen Instruments erfasst. Darin enthaltene Bilddaten werden mit in entsprechenden Datensätzen gespeicherten Identifikationsmerkmalen abgeglichen. Anhand eines in dem Bild vorhandenen Identifikationsmerkmals wird ein Typ des zu reinigenden chirurgischen Instruments identifiziert. Auf der Projektionsfläche werden dann visuelle Informationen betreffend Anweisungen zur Reinigung dieses Typs angezeigt.

Aus der US 2015/0248170 A1 sind ein Verfahren und ein System zum Generieren eines virtuellen Benutzerinterfaces in Relation zu einem Totem bekannt. Dabei wird basierend auf einer detektierten Manipulation des Totems das virtuelle Benutzerinterface erzeugt. Das virtuelle Benutzerinterface wird an aus einem virtuellen Weltmodell bestimmten Kartenpunkten gerendert, welche mit einer Position des Toten verknüpft sind, sodass das Benutzerinterface aus Sicht eines Benutzers stationär an der Position des Totems erscheint.

Die US 2014/002337 A1 beschreibt eine Pistole zur Projektion einer Bedienoberfläche auf einer Darstellungsfläche mit einer maschinenlesbaren Kennzeichnung, welche ein Layout und/oder eine Funktionsauswahl der Bedienoberfläche kodiert.

Aufgabe der vorliegenden Erfindung ist es, eine verbesserte erstfehlersichere Bedienung eines medizinischen Geräts zu ermöglichen.

Diese Aufgabe wird erfindungsgemäß durch die Gegenstände der unabhängigen Patentansprüche gelöst. Vorteilhafte Ausgestaltungen und Weiterbildungen der Erfindung sind in den abhängigen Patentansprüchen sowie in der Beschreibung und in den Zeichnungen angegeben.

Eine erfindungsgemäße Bedienvorrichtung für ein medizinisches Gerät umfasst eine Projektionseinrichtung, welche zur reellen und/oder virtuellen Projektion einer Bedienoberfläche auf ein Darstellungselement eingerichtet ist, sowie eine Erfassungseinrichtung zum berührungslosen Erfassen einer Interaktion einer Bedienperson mit der projizierten Bedienoberfläche. Bereits eine derartige Bedienvorrichtung kann gegenüber einer herkömmlichen Bedienung eine verbesserte Bedienung eines medizinischen Gerätes, insbesondere in einem sterilen Umfeld, ermöglichen. Um eine solche verbesserte Bedienung des medizinischen Geräts auch in Situationen und Anwendungsfällen zu ermöglichen, in denen eine Erstfehlersicherheit gefordert ist, ist es erfindungsgemäß vorgesehen, dass an dem Darstellungselement eine von der Erfassungseinrichtung separate Sensorik zum unabhängigen Erfassen der Interaktion angeordnet ist. Mit anderen Worten ist es also vorgesehen, dass die Interaktion, also beispielsweise eine Bedienhandlung der Bedienperson, auf zumindest zwei voneinander unabhängigen Wegen, insbesondere auf zwei unterschiedliche Arten, erfasst oder detektiert wird. Durch diese redundante Erfassung oder Detektion der Interaktion ist eine Absicherung gegen eine versehentliche Bedienung oder Fehlbedienung und/oder gegen eine Fehlerkennung der beziehungsweise einer Interaktion gegeben. Dadurch kann vorteilhaft, insbesondere in sicherheitsrelevanten oder sicherheitskritischen Situationen, eine Bedien- und Patientensicherheit verbessert beziehungsweise sichergestellt werden. Gleichzeitig bietet die Bedienvorrichtung dabei Bedienmöglichkeiten, welche gegenüber einer herkömmlichen, beispielsweise auf einer Betätigung von physischen Tasten oder Hebeln basierenden Bedienung, besonders flexibel, insbesondere auch dynamisch beziehungsweise situationsabhängig, anpassbar sowie besonders anschaulich und intuitiv besonders leicht erfassbar sind. Zudem ermöglicht die erfindungsgemäße Bedienvorrichtung besonders vorteilhaft mit deutlich geringerem Aufwand eine verbesserte Einhaltung von Anforderungen bezüglich einer Sterilität eines Arbeitsumfeldes beziehungsweise von Berührungspunkten zwischen der Bedienperson, beispielsweise einem behandelnden Arzt, und dem medizinischen Gerät.

Beispielsweise kann aus Sicherheitsgründen eine Gerätebewegung, welche potentiell zu einer Kollision mit einem Patienten führen kann, allein durch eine Gestensteuerung unzulässig sein, um eine Gefährdung des Patienten zu vermeiden. Hier kann also zur Freigabe oder Bestätigung der Gerätebewegung zusätzlich zu der Bediengeste ein separates Signal, das heißt eine separate Interaktion, notwendig sein.

Das Darstellungselement ist im Sinne der vorliegenden Erfindung ein physisches, reales Objekt, beispielsweise in Form einer Platte. Eine Oberfläche oder ein Oberflächenbereich, also beispielsweise eine Seite oder Seitenfläche, des Darstellungselements bildet eine Projektionsfläche, auf welcher die Bedienoberfläche dargestellt wird und/oder zumindest für die Bedienperson, das heißt aus Sicht der Bedienperson, erscheint. Die Bedienoberfläche erscheint also zumindest für die Bedienperson oder aus Sicht der Bedienperson dem Darstellungselement überlagert. Bei einer reellen Projektion der Bedienoberfläche kann diese beispielsweise auch von weiteren Personen ohne technische Ausrüstung gesehen werden. Bei einer virtuellen Projektion kann die Bedienoberfläche beispielsweise lediglich virtuell, das heißt also scheinbar, auf dem Darstellungselement dargestellt werden oder erscheinen und ist dann nur für die Bedienperson sichtbar. Hierfür kann beispielsweise die Projektionseinrichtung eine Datenbrille oder ein Teil einer Datenbrille oder eines vergleichbaren Gerätes sein. Dabei kann die Bedienoberfläche real also beispielsweise mittels eines transparenten oder teiltransparenten Elements, beispielsweise einer Sichtscheibe, der Projektionseinrichtung - beziehungsweise der Datenbrille - derart angezeigt werden, dass sie für die die Projektionseinrichtung tragende oder verwendende Bedienperson stationär auf oder an dem Darstellungselement erscheint. Es ist im Falle einer virtuellen Projektion also möglich, dass umstehende weitere Personen die Bedienoberfläche auf dem Darstellungselement nicht sehen können, zumindest sofern sie nicht ebenfalls eine entsprechende Projektionseinrichtung verwenden.

Insbesondere bei einer virtuellen Projektion der Bedienoberfläche handelt es sich also um eine augmentierte-Realität-Anwendung (augmented-reality oder mixed-reality-projection). Es kann auch möglich sein, die Bedienoberfläche gleichzeitig reell und virtuell auf das Darstellungselement zu projizieren. Dies hat den Vorteil, dass die Bedienoberfläche sowohl für die Bedienperson als auch für weitere Personen sichtbar ist, welche so beispielsweise besonders schnell und einfach durch die dargestellte Bedienoberfläche informiert werden können. Zudem kann so auch eine besonders flexible Bedienbarkeit der Bedienvorrichtung durch mehrere Personen besonders einfach realisiert werden. Hierfür kann beispielsweise zumindest eine weitere Erfassungseinrichtung vorgesehen sein, welche eine Interaktion einer weiteren Person mit der reell projizierten Bedienoberfläche erfasst. Dabei kann also die Bedienperson mit der virtuell dargestellten Bedienoberfläche interagieren und die weitere Person kann mit der reell projizierten Bedienoberfläche interagieren. In diesem Fall können bevorzugt mehrere Erfassungseinrichtungen vorgesehen sein. Ebenso kann es möglich sein, dass sowohl die Bedienperson als auch die weitere Person mit der reell projizierten Bedienoberfläche interagieren, wobei dann eine Erfassungseinrichtung ausreichen kann. Ebenso kann es möglich sein, dass sowohl die Bedienperson als auch die zumindest eine weitere Person jeweils eine eigene Projektionseinrichtung, insbesondere eine eigene Datenbrille, verwenden, wobei dann jeweilige virtuelle Projektionen untereinander synchronisiert sein können. Die jeweiligen virtuellen Projektionen stellen dabei die Bedienoberfläche in jeweils individuell korrekter blickwinkel- und positionsabhängiger Perspektive dar. In diesem Fall kann etwa eine besonders gute Zusammenarbeit mehrerer Personen verbessert werden. Insbesondere können auch synchronisierte Darstellungen der Bedienoberfläche auf unterschiedlichen Darstellungselementen erzeugt werden, sodass auch eine Zusammenarbeit von Personen ermöglicht wird, welche sich nicht im selben Raum aufhalten. Mögliche Anwendungsgebiete synchronisierter Darstellungen der Bedienoberfläche umfassen sind in den Bereichen Telemedizin, Training oder Schulung und Service gegeben.

Die Erfassungseinrichtung zum berührungslosen Erfassen der Interaktion kann beispielsweise zumindest eine Kamera, bevorzugt eine stereoskopische oder 3D-Kamera, und/oder weitere Sensoren sowie zumindest eine zur Verarbeitung beziehungsweise Auswertung entsprechender Kamera- und/oder Sensordaten eingerichtete Auswerteeinrichtung umfassen. Insbesondere kann das berührungslose Erfassen eine Gestenerkennung einschließen. Alternativ oder zusätzlich kann die Erfassungseinrichtung einen von der Bedienperson getragenen oder an der Bedienperson angeordneten Sensor, insbesondere zur Bewegungsund/oder Gestenerfassung, umfassen. Dazu kann beispielsweise ein Beschleunigungs- und/oder ein Lagesensor verwendet werden. Ebenso ist alternativ oder zusätzlich eine Positionsund/oder Bewegungserfassung und/oder -verfolgung, insbesondere eine Gestenerfassung, beispielsweise mittels eines Radarsensors möglich. Die Erfassungseinrichtung ist also nicht auf optische und/oder passive Erfassungsmethoden beschränkt. Ebenso kann die Erfassungseinrichtung zumindest einen elektrischen Sensor, beispielsweise einen Elektromyographiesensor, umfassen. Ebenso kann die Erfassungseinrichtung einen, beispielsweise kapazitiven, Annäherungssensor umfassen, mittels welchem eine Lage, Position, Bewegung und/oder Geste der Bedienperson erfassbar ist. Die Erfassungseinrichtung oder ein Sensor der Erfassungseinrichtung kann an oder in dem Darstellungselement angeordnet sein. Die Verwendung eines derartigen, insbesondere nicht auf eine rein optische Erfassung angewiesenen, Sensors oder einer entsprechenden Erfassungseinrichtung kann gerade im medizinischen Umfeld besonders vorteilhaft sein, da hier oftmals die Bedienperson nicht frei ist in der Wahl ihrer Position oder Haltung - beispielsweise während einer Untersuchung oder Behandlung eines Patienten. Es kann also die Gefahr minimiert werden, dass eine Geste aufgrund eines ungünstigen Erfassungswinkels einer Kamera nicht erfasst wird. Zudem können derartige Sensoren auch kleinste Bewegungen erfassen, was beispielsweise bei oftmals beschränkten Platzverhältnissen in einer Untersuchungs- oder Behandlungssituation besonders vorteilhaft sein kann.

Die separate Sensorik zum unabhängigen Erfassen der Interaktion kann gleich oder ähnlich wie die Erfassungseinrichtung aufgebaut sein, wobei die Sensorik und die Erfassungseinrichtung die Interaktion, beispielsweise eine Geste der Bedienperson, dann aus unterschiedlichen Blick-, Betrachtungs- oder Erfassungswinkeln detektieren können. Dadurch kann die Gefahr einer Fehlerkennung oder Fehlinterpretation der Interaktion, beispielsweise aufgrund einer für die Erkennung und Auswertung ungünstigen Perspektive der Erfassungseinrichtung oder der Sensorik, signifikant reduziert werden.

Um einen Bauteil- und Kostenaufwand gering zu halten, die Sterilität der Bedienvorrichtung möglichst einfach sicherstellen und aufrecht erhalten zu können und die Erstfehlersicherheit weiter zu verbessern, ist es jedoch bevorzugt vorgesehen, dass die Erfassungseinrichtung und die Sensorik auf unterschiedlichen Funktionsprinzipien zur Erfassung oder Detektion der Interaktion basieren. Es ist dabei nicht zwingend notwendig, dass die Interaktion von der Erfassungseinrichtung und der Sensorik in gleicher Auflösung oder in gleichem Detailgrad erfasst wird. So kann es beispielsweise bereits ausreichen, dass die Sensorik dazu eingerichtet ist, zu erfassen oder zu detektieren, ob die Bedienperson mit der Bedienoberfläche oder der Bedienvorrichtung interagiert hat, ob also überhaupt eine Bedienhandlung erfolgt ist.

Es ist möglich, zumindest einen Teil der Bedienoberfläche und/oder eine zweite Bedienoberfläche und/oder weitere Bedienelemente für die Bedienperson unabhängig von dem Darstellungselement rein virtuell zu projizieren oder anderweitig darzustellen. Diese Projektion oder Darstellung kann beispielsweise virtuell im freien Raum schwebend angezeigt werden. Es kann sich dabei auch um ein Hologramm oder eine hologrammartige Projektion handeln.

In bevorzugter Ausgestaltung der vorliegenden Erfindung umfasst das Darstellungselement ein Oberteil und ein Unterteil, wobei das Unterteil die Sensorik umfasst und das Oberteil beschädigungsfrei und reversibel von dem Unterteil lösbar an diesem gehalten ist. Mit anderen Worten ist dann das Darstellungselement also zumindest zweiteilig ausgebildet, wobei beide Teile voneinander trennbar sind. Insbesondere weist das von dem Unterteil abnehmbare Oberteil keinerlei, zumindest keinerlei temperaturempfindliche, Elektrik, Elektronik oder Schaltungen auf. Dies hat den in einem medizinischen, insbesondere in einem sterilen, Umfeld entscheidenden Vorteil, dass das Oberteil besonders einfach, sicher und beschädigungsfrei sterilisierbar ist. Besonders bevorzugt kann das Oberteil dazu aus einem beschädigungsfrei dampfsterilisierbaren und/oder autoklavierbaren Werkstoff, insbesondere einem Kunststoff, einem Glas, einem keramischen oder metallischen Werkstoff, gebildet sein. Somit kann also das Oberteil auch einzeln, das heißt unabhängig oder getrennt von dem Unterteil und dem medizinischen Gerät sterilisiert werden. Dadurch kann eine keimfreie Bedienbarkeit des medizinischen Gerätes besonders einfach sichergestellt werden, da das Oberteil als Darstellung- oder Projektionsfläche für die Bedienoberfläche dienen und somit den einzigen Berührungspunkt zwischen der Bedienperson und der Bedienvorrichtung beziehungsweise dem medizinischen Gerät während dessen Bedienung bilden kann. Das Darstellungselement, insbesondere das Oberteil kann beispielsweise eine Größe wie ein heutzutage üblicher mobiler, also tragbarer, Tabletcomputer haben. Das Oberteil kann beispielsweise mittels einer magnetischen Verbindung oder Halterung an dem Unterteil gehalten sein. Ebenso kann es möglich sein, eine Verrastung, eine Klemmung, eine geometrisch ausgeformte Aufnahme oder Halterung oder eine sonstige form- oder kraftschlüssige Verbindung zum Halten des Oberteils an dem Unterteil vorzusehen.

Das Oberteil kann bevorzugt an die Funktionalität der Sensorik und/oder der Erfassungseinrichtung angepasst sein. Dazu kann das Darstellungselement beispielsweise ganz oder teilweise transparent, nichtleitend und/oder antimagnetisch ausgebildet und/oder hinsichtlich seiner Reflektivität angepasst sein. Dadurch kann vorteilhaft eine Funktionsweise der Sensorik und/oder der Erfassungseinrichtung unterstützt werden. Besonders vorteilhaft kann das Oberteil durchlässig für von der in oder an dem Unterteil angeordneten Sensorik verwendete Strahlung, beispielsweise Radiowellen und/oder Terahertzwellen, ausgebildet sein. Entsprechendes kann allgemein für das Darstellungselement, beispielsweise auch bei einteiliger Gestaltung, gelten.

Besonders vorteilhaft kann das Darstellungselement, insbesondere das Oberteil, eine antimikrobielle Beschichtung aufweisen.

Das Oberteil kann - etwa durch eine ausreichende Materialdicke - formstabil ausgebildet sein, um eine Handhabung zu vereinfachen und eine Beschädigungsgefahr zu minimieren. Alternativ kann das Oberteil als Folie ausgebildet sein. Dadurch können vorteilhaft Material, Gewicht und Bauraum eingespart werden. Zudem kann hierdurch gegebenenfalls eine Dämpfung der von der Sensorik und/oder der Erfassungseinrichtung verwendeten Strahlung vorteilhaft minimiert werden. Die Ausbildung des Oberteils als Folien kann vorteilhaft auch eine besonders flexible Anordenbarkeit ermöglichen. Dabei kann es möglich sein, das Oberteil mittels einer Unterdruckhalterung zu halten.

Durch die zumindest zweiteilige Gestaltung beziehungsweise den zumindest zweiteiligen Aufbau des Darstellungselements und die Anordnung der Sensorik in dem - bei einem bestimmungsgemäßen Betrieb bevorzugt an einer von der Bedienperson abgewandten Seite des Oberteils angeordneten - Unterteil wird es besonders vorteilhaft beispielsweise ermöglicht, auf eine Abdeckung des Oberteils mit einer sterilen durchsichtigen Folie zu verzichten, wie sie bei herkömmlichen Bedienteilen medizinischer Geräte heutzutage typischerweise verwendet wird. Durch diese sterile Abdeckung werden eine jeweilige Bedienergonomie und/oder eine Erkennbarkeit jeweiliger Bedienelemente erheblich eingeschränkt und die Anbringung der Abdeckung erfordert zusätzliche, zeitaufwendige Arbeitsschritte beispielsweise bei einer Vorbereitung eines Operationssaals. Diese Nachteile können durch die vorliegende Erfindung umgangen werden. So kann beispielsweise das medizinische Gerät samt des an diesem angeordneten Unterteils sehr einfach mit einer sterilen Abdeckung oder Umhüllung umgeben werden, während das Oberteil separat sterilisiert und trotz der Abdeckung des Unterteils an diesem gehalten werden kann. Die beiden Teile können dabei trotz der zwischen ihnen angeordneten Abdeckung problemlos aneinander gehalten werden. Es ist aber auch möglich, beispielsweise ein relativ kleines ein Loch in der Abdeckung vorzusehen, durch welche eine die beiden Teile verbindende Halterung hindurchtreten kann, ohne dass dadurch die Sterilität des Oberteils oder des jeweiligen Arbeitsbereiches signifikant gefährdet würde.

In bevorzugter Ausgestaltung der vorliegenden Erfindung ist es vorgesehen, dass die Sensorik dazu eingerichtet ist, die Interaktion mittels einer Auswertung einer Berührung des Darstellungselements durch die Bedienperson zu erfassen. Mit anderen Worten ist es also vorgesehen, dass die Bedienperson das Darstellungselement berührt beziehungsweise berühren muss, um eine Interaktion mit der Bedienoberfläche vorzunehmen beziehungsweise die Erfassung oder Detektion dieser Interaktion durch die Sensorik zu bewirken oder sicherzustellen. Hierdurch kann besonders vorteilhaft eine versehentliche Bedienung der Bedienvorrichtung, beispielsweise basierend auf einer unbeabsichtigt als Interaktion oder Bediengeste von der Erfassungseinrichtung erfassten Geste oder Bewegung, vermieden werden. Um die Berührung des Darstellungselements auswerten zu können, ist die Sensorik dazu eingerichtet, die Berührung zu erfassen oder zu detektieren. Beispielsweise kann die Sensorik einen Sensor umfassen, welcher eine durch die Berührung verursachte Bewegung des Darstellungselements, einen durch die Berührung von der Bedienperson ausgeübten Druck auf das Darstellungselement und/oder eine mit der Berührung, der Bewegung oder dem Druck einhergehende Änderung beispielsweise einer elektrischen Größe, wie etwa einer Kapazität, einer Induktivität oder eines elektrischen Widerstands, sensiert. Die Sensorik kann also beispielsweise einen bewegungsempfindlichen Sensor, einen Drucksensor, einen kapazitiven Sensor, einen Dehnungsmessstreifen oder dergleichen umfassen. Ebenso können mehrere derartige Sensoren vorgesehen sein.

Durch die Verwendung mehrerer Sensoren und/oder durch die Verwendung unterschiedlicher Sensoren kann vorteilhaft eine Redundanz für die Erfassung der Interaktion mittels der Sensorik geschaffen werden, wodurch eine Zuverlässigkeit, eine Ausfallsicherheit und eine Bediensicherheit der Bedienvorrichtung verbessert werden können. Hier kann also ein Grundprinzip der vorliegenden Erfindung, nämlich eine wenigstens zweikanalige Messung, auf untergeordneter Ebene angewendet werden. Die Sensorik beziehungsweise zumindest ein Sensor der Sensorik kann beispielsweise an einer Seite oder Halterung des Darstellungselements angeordnet sein, welche bevorzugt der Bedienperson beziehungsweise einer als reale oder virtuelle Projektionsfläche dienenden Seite oder Oberfläche des Darstellungselements abgewandt ist. Hierdurch kann vorteilhaft sichergestellt werden, dass diese der Projektion dienende Fläche sicher sterilisierbar ist, ohne die Sensorik zu beschädigen. Ebenso wird vermieden, dass die Darstellung der Bedienoberfläche bei einer reellen Projektion durch die Sensorik in ihrer Erkennbarkeit eingeschränkt oder beeinträchtigt wird.

Bevorzugt kann die Sensorik oder der zumindest eine Sensor der Sensorik an einer Verbindung oder Halterung zwischen dem Oberteil und dem Unterteil angeordnet oder in diese Verbindung oder Halterung integriert sein oder diese Verbindung oder Halterung bilden. Hierdurch kann vorteilhaft eine besonders hohe Sensitivität für die Berührung erzielt werden, da der entsprechende Druck oder die entsprechende Bewegung des Oberteils unmittelbar auf den Sensor einwirkt oder über diesen an das Unterteil weiter gegeben beziehungsweise an diesem abgestützt wird.

In bevorzugter Ausgestaltung der vorliegenden Erfindung ist die Bedienoberfläche in mehrere Sektoren, beispielsweise Quadranten, unterteilt, wobei die Sensorik mehrere verteilt angeordnete Sensoren sowie eine Auswerteeinrichtung umfasst, welche dazu eingerichtet ist, mittels einer Auswertung von Sensordaten der einzelnen Sensoren zu bestimmen, in welchem Sektor die Interaktion erfolgt beziehungsweise erfolgt ist. Es können also beispielsweise die jeweiligen Sensordaten oder Sensorsignale miteinander verglichen werden, um zu bestimmen, mit welchem Teil des Darstellungselements und dementsprechend mit welchem Teil der Bedienoberfläche die Bedienperson interagiert beziehungsweise interagiert hat. Hierdurch kann die Erstfehlersicherheit der Bedienvorrichtung weiter verbessert werden, da die Sensorik nunmehr nicht nur erfassen kann, ob überhaupt eine Interaktion stattgefunden hat, sondern beispielsweise welche von mehreren unterschiedlichen Funktionen durch die Interaktion ausgelöst werden soll beziehungsweise in welchem Sektor, Segment oder Teilbereich der Bedienoberfläche und/oder des Darstellungselements die Interaktion stattgefunden hat. Somit wird also ein genauerer Abgleich zwischen den von der Erfassungseinrichtung und der Sensorik erfassten Interaktionen ermöglicht, wodurch mögliche Diskrepanzen mit höherem Detailgrad, also mit verbesserter Genauigkeit erkannt werden können.

Beispielsweise kann es dazu vorgesehen sein, dass an vier Eckbereichen des Darstellungselements je ein Drucksensor angeordnet ist. Berührt dann die Bedienperson das Darstellungselement in einem dieser Eckbereiche, so wird der dort angeordnete Drucksensor einen relativ hohen Druck messen, während beispielsweise ein diagonal gegenüber angeordneter Drucksensor einen relativ geringen Druck messen wird. Bei einer Berührung in einem mittigen oder zentralen Bereich des Darstellungselements, welcher gleiche Abstände zu allen vier Drucksensoren aufweist, werden alle vier Drucksensoren einen, zumindest im Wesentlichen, gleichen Druck messen. Auf diese Art und Weise kann aus einem Vergleich der Sensordaten mehrerer Sensoren eine Position ermittelt werden, an welcher die Bedienperson das Darstellungselement berührt, das heißt an der die Interaktion stattfindet oder stattgefunden hat. Vorteilhaft kann mittels der Sensorik so erkannt werden, mit welcher Funktion, das heißt mit welchem Teil oder welchem Bedienelement der Bedienoberfläche, die Bedienperson interagiert hat, wenn jedem Sektor genau eine Funktion oder ein Bedienelement zugeordnet ist.

Es kann jedoch auch vorteilhaft sein, einem oder mehreren Sektoren der Bedienoberfläche mehrere Funktionen zuzuordnen. Dabei können bevorzugt gleichartige, das heißt beispielsweise inhaltlich, thematisch oder bezüglich ihrer Auswirkung auf das medizinische Gerät oder einen Patienten ähnliche Funktionen in jeweils einem Sektor gebündelt oder zusammengefasst sein. Jedem Sektor kann also eine bestimmte Klasse oder Kategorie von Funktionen zugeordnet sein. Hierdurch kann vorteilhaft eine besonders zuverlässige Absicherung dahingehend ermöglicht werden, ob die mittels der Erfassungseinrichtung erfasste Interaktion zumindest hinsichtlich der Kategorie oder Klasse der entsprechenden Funktion mit der mittels der Sensorik erfassten Interaktion übereinstimmt.

Alternativ kann es vorteilhaft sein, ähnliche Funktionen unterschiedlichen Sektoren zuzuordnen. Hierdurch kann eine besonders genaue Identifizierung und somit auch Absicherung gegenüber einer Fehlerkennung der Interaktion durch die Erfassungseinrichtung ermöglicht werden, insbesondere dann, wenn zum Auslösen dieser ähnlichen Funktionen beispielsweise ähnliche Gesten vorgesehen sind.

Vorteilhaft kann die Benutzer- oder Bedienoberfläche entsprechend einem vorgegebenen Raster unterteilt oder aufgebaut sein, sodass beispielsweise einzelne Bedienelemente immer an vorgegebenen Positionen oder Relativpositionen dargestellt werden. Zudem kann das Darstellungselement physisch ausgeformte Fräsungen, Erhebungen, Vertiefungen, Riffelungen und/oder sonstige Oberflächenmerkmale aufweisen, welche ebenfalls an dem vorgegebenen Raster oder Anordnungsmuster der virtuellen oder projizierten Bedienelemente der Bedienoberfläche ausgerichtet sein können. Auf diese Art und Weise kann einer jeweiligen Bedienperson ermöglicht werden, nur virtuell oder projiziert dargestellte Bedienelemente oder Funktionsbereiche der Bedienoberfläche physisch zu ertasten oder zu erfühlen, wodurch eine besonders sichere und zuverlässige Bedienung ermöglicht werden kann.

In bevorzugter Ausgestaltung der vorliegenden Erfindung ist die Sensorik mit einer Aktuatorik kombiniert, welche dazu eingerichtet ist, als Reaktion auf eine, insbesondere mittels der Sensorik, erfasste Interaktion ein haptisches Signal an dem Darstellungselement zu erzeugen. Mit anderen Worten kann also ein haptisches Feedback vermittelt werden, wodurch die jeweilige Bedienperson besonders genau und zuverlässig erkennen kann, ob sie die Interaktion erfolgreich durchgeführt hat beziehungsweise ob die Interaktion erfolgreich erfasst worden ist. Dies kann beispielsweise besonders vorteilhaft sein bei Funktionen, welche nicht unmittelbar sichtbare Auswirkungen haben, welche also beispielsweise eine Steuerung unsichtbarer Strahlung betreffen.

Gerade in einem medizinischen Umfeld kann die Erzeugung des haptischen Signals besonders vorteilhaft sein, da jeweilige Bedienpersonen oftmals Handschuhe tragen, wodurch gegebenenfalls eine taktile Wahrnehmungsfähigkeit eingeschränkt sein kann. Es kann möglich sein, als haptisches Signal beispielsweise eine Vibration des Darstellungselements, insbesondere des Oberteils, zu erzeugen. Um eine noch klarere und eindeutigere haptische Rückmeldung an die jeweilige Bedienperson zu geben, kann es vorgesehen sein, je nach betätigter Funktion beziehungsweise je nach erfasster Interaktion oder Interaktionsart ein unterschiedliches haptisches Signal zu erzeugen. Das haptische Signal kann also in Abhängigkeit von der Interaktion erzeugt werden. Dadurch erhält die jeweilige Bedienperson also unmittelbar eine Rückmeldung darüber, ob sie die jeweils gewünschte Funktion erfolgreich bedient oder ausgelöst hat. Unterschiedliche haptische Signale können sich beispielsweise in ihrer Länge, ihrer Frequenz, einem zeitlichen Muster oder einer Stärke oder Amplitude unterscheiden.

In vorteilhafter Ausgestaltung der vorliegenden Erfindung umfasst die Projektionseinrichtung ein Head-Mounted Display (HMD), insbesondere eine augmented-reality-Brille.

Der Begriff "Projektionseinrichtung" ist im Sinne der vorliegenden Erfindung breit auszulegen und soll eine Einrichtung bezeichnen, welche zur Darstellung der Bedienoberfläche geeignet und eingerichtet ist. Bei dieser Darstellung muss es sich also nicht zwangsläufig um eine Projektion im strengen Wortsinne handeln. Ebenso kann es beispielsweise vorgesehen sein, dass zumindest ein Teilbereich eines Sichtglases des HMD als, insbesondere transparenter oder teiltransparenter, Bildschirm ausgebildet ist. Ebenso kann eine der Darstellung der Bedienoberfläche dienende Lichteinkopplung beispielsweise über einen oder mehrere Lichtleiter vorgesehen sein.

Die Verwendung eines HMD ist besonders vorteilhaft, da hierdurch eine Bewegungs- und Handlungsfreiheit der Bedienperson nicht eingeschränkt wird. Zudem kann durch die Verwendung des HMD in Verbindung mit der erfindungsgemäßen Bedienvorrichtung sichergestellt werden, dass sämtliche benötigten Funktionen und Bedienelemente an einem einzigen Ort zur Verfügung stehen, wodurch eine besonders einfache, schnelle und effiziente Bedienung ermöglicht wird. Besonders vorteilhaft ist dabei, dass durch projizierte, das heißt also letztlich elektronisch erzeugte, Bedienoberfläche beispielsweise nur eine Auswahl von situationsabhängig benötigten Funktionen und Bedienelementen dargestellt werden kann. Somit stellt eine begrenzte Größe des Darstellungselements effektiv keine Beschränkung dar, da nicht notwendigerweise gleichzeitig sämtliche möglichen Funktionen oder Bedienelemente zugänglich sein, also dargestellt werden müssen.

Besonders bevorzugt wird durch die Verwendung des HMD eine Vielzahl weiterer Bedienmöglichkeiten ermöglicht, welche gerade in einem sterilen Umfeld besonders vorteilhaft für eine berührungslose Bedienung, insbesondere medizinischer Geräte, nutzbar sind. Dazu kann es bevorzugt vorgesehen sein, dass - beispielsweise ebenfalls mittels der Erfassungseinrichtung - das jeweils zu bedienende Gerät erkannt und im Raum eindeutig lokalisiert wird. Dazu kann also beispielsweise eine 3D-Kamera verwendet werden. Diese kann Teil des HMD, an diesem angeordnet oder von diesem separat beispielsweise an einer Wand oder Decke des jeweiligen Raumes angeordnet sein. Durch eine Anordnung an dem oder als Teil des HMD kann vorteilhaft sichergestellt werden, dass sich das jeweils zu bedienende Gerät im Blickfeld der Bedienperson befindet. Hierdurch kann vorteilhaft eine dynamische Anpassung der Bedienoberfläche und/oder der zur Verfügung stehenden Bedienfunktionen oder Bediengesten erfolgen, da diese jeweils auf das im Blickfeld der Bedienperson befindliche zu bedienende Gerät abgestimmt werden können. Eine Anordnung der Erfassungseinrichtung und/oder einer weiteren Einrichtung, beispielsweise der 3D-Kamera, an einer ortsfesten Einrichtung, beispielsweise einer Raumwand oder -decke, kann vorteilhaft eine besonders gute Erfassbarkeit sowohl der Bedienperson als auch aller bedienten Geräte ermöglichen, ohne dass es zu perspektivischen Verdeckungen kommt.

Ebenso kann es möglich sein, die Aktuatorik der Sensorik oder eine weitere Aktuatorik ganz oder teilweise in oder an dem HMD anzuordnen. Damit kann ein haptisches Signal an einem Kopf der Bedienperson erzeugt werden. Die Anordnung in oder an dem HMD kann vorteilhaft eine besonders einfache Ausgestaltung des Darstellungselements und dabei weiterhin ein haptisches Feedback ermöglichen. Besonders vorteilhaft kann diese Anordnung auch sein, da es hierdurch ermöglicht wird, mehreren Personen, welche jeweils ein eigenes HMD verwenden, das haptische Signal zu übermitteln. Somit können also mehrere Personen simultan jeweilige Bedienhandlungen der Bedienperson mitverfolgen und nachvollziehen, wodurch etwa eine Zusammenarbeit und/oder Schulung verbessert werden kann. Auch kann so eine Immersion von entfernt befindlichen teilnehmenden Personen in einen jeweiligen mittels augmentierter oder virtueller Realität vermittelten Vorgang verbessert werden.

Erfindungsgemäß ist es vorgesehen, dass das Darstellungselement eine maschinenlesbare Kennzeichnung, insbesondere einen QR-Code umfasst, welche einen Typ des Darstellungselements angibt. Die Bedienvorrichtung ist dann dazu eingerichtet, diese Kennzeichnung zu lesen, das heißt zu erfassen und auszuwerten, wofür beispielsweise ebenfalls die Erfassungseinrichtung verwendbar sein kann. Die Projektionseinrichtung ist dann dazu eingerichtet, die zu projizierende Bedienoberfläche in Abhängigkeit von dem Typ des Darstellungselements aus einer Vielzahl von vorgegebenen unterschiedlichen Bedienoberflächen auszuwählen. Mit anderen Worten ist also an dem Darstellungselement eine physische Markierung vorgesehen, welche eine Erkennung oder Identifizierung des Darstellungselementes und/oder einer oder mehrerer Eigenschaften des Darstellungselementes durch die Bedienvorrichtung ermöglicht. Der Typ des Darstellungselements kann durch eine oder mehrere Eigenschaften des Darstellungselements bestimmt sein. Dies kann beispielsweise eine Größe, einen vorgesehenen Einsatzzweck und/oder Einsatzort, eine Spezifikation der jeweiligen Sensorik, eine Farbe und/oder dergleichen mehr umfassen. Ebenso kann durch die Kennzeichnung beispielsweise angegeben werden, wo, insbesondere an welchem Gerät, sich das Darstellungselement befindet, das heißt insbesondere zur Bedienung welchen Gerätes das Darstellungselement vorgesehen ist. Durch die Verwendung einer solchen expliziten und dedizierten maschinenlesbaren Kennzeichnung kann besonders vorteilhaft die Erkennung und Identifizierung des Darstellungselementes als solches durch die Bedienvorrichtung unterstützt beziehungsweise verbessert werden. Zudem kann die Bedienoberfläche beziehungsweise deren Darstellung besonders genau und zuverlässig in Abhängigkeit von der Kennzeichnung beziehungsweise in Abhängigkeit von durch die Kennzeichnung kodierten Daten, an das jeweilige Darstellungselement angepasst werden. Besonders anschaulich ist dies beispielsweise dann möglich, wenn die Kennzeichnung die Größe und Farbe des Darstellungselements beziehungsweise einer zur Darstellung der Bedienoberfläche vorgesehenen Fläche des Darstellungselements kodiert ist. Vorteilhaft kann beispielsweise auch eine Position dieser Fläche relativ zur Position der Kennzeichnung durch diese kodiert oder vermittelt werden. Durch Auswertung der Kennzeichnung kann dann die Bedienoberfläche optimal an das jeweilige Darstellungselement angepasst skaliert sowie hinsichtlich eines verwendeten Farbschemas angepasst werden, um eine optimale Darstellung und Erkennbarkeit sicherzustellen.

Alternativ zu einem QR-Code können auch andere Kennzeichnungen, wie beispielsweise ein Lochmuster, ein Strichcode oder dergleichen verwendet werden.

Ebenso kann die Kennzeichnung beispielsweise eine Softwarelizenz oder eine entsprechende Verwendungsberechtigung für ein Softwareprodukt und/oder ein Gerät kodieren beziehungsweise auf entsprechende Daten verweisen. Letzteres kann etwa durch Kodierung einer Adresse eines entsprechenden Datensatzes auf einem Lizenzserver erfolgen. Somit kann es also beispielsweise vorgesehen sein, dass die Bedienoberfläche nur durch die jeweilige Lizenz oder Berechtigung freigegebene Funktionen und/oder Bedienelemente umfasst oder zugänglich macht.

Vorteilhaft kann die Kennzeichnung austauschbar sein. Beispielsweise kann es vorgesehen sein, dass die Bedienperson oder ein entsprechender Anwender die Kennzeichnung, also beispielsweise den QR-Code, selbst erstellt und/oder selbst an dem Darstellungselement anbringt. Die Kennzeichnung kann beispielsweise angeklebt, magnetisch oder durch Klemmung gehalten oder angeschraubt werden. Die Bedienperson kann also beispielsweise die Kennzeichnung beziehungsweise den QR-Code rechnergestützt erzeugen und dann beispielsweise ausdrucken und auf das Darstellungselement aufkleben.

Vorliegend wird durch die Kennzeichnung ein Layout und/oder eine Funktionsauswahl der Bedienoberfläche kodiert. Daraus ergibt sich die vorteilhafte Möglichkeit, dass die Bedienoberfläche oder zumindest eine Eigenschaft der Bedienoberfläche von der Bedienperson individuell vorgebbar ist. So kann die Bedienperson - etwa mittels eines Editors -beispielsweise eine Auswahl und Anordnung von Bedienelementen und/oder eine Farbe der Bedienoberfläche bedarfsgerecht bestimmen. Besonders vorteilhaft können derartige Konfigurationen dabei vorab erstellt und durch Austausch der Kennzeichnung an dem Darstellungsgerät besonders schnell gewechselt werden. Besonders vorteilhaft kann dies beispielsweise während einer Behandlung durchgeführt werden, ohne dass dabei ein gegebenenfalls unsteriles Bediengerät verwendet werden müsste.

Eine derartige maschinenlesbare Kennzeichnung kann beispielsweise auch an dem jeweils zu bedienenden Gerät angebracht oder angeordnet sein, wodurch besonders vorteilhaft eine Erkennung, Identifizierung und Lokalisierung des Geräts oder bestimmter Teilelemente des Geräts unterstützt wird. So kann beispielsweise die Bedienung des Geräts mittels Gesten und entsprechender Gestenerkennung verbessert werden, indem zusätzlich zu den Gesten auch die in der oder den jeweiligen Kennzeichnungen enthaltenen Daten bei der Interpretation oder Auswertung der Gesten, das heißt also zur Steuerung oder Bedienung des Geräts, ausgewertet beziehungsweise verwendet oder berücksichtigt werden. Somit wird vorteilhaft besonders zuverlässig eine Bedienung von Geräten ermöglicht, ohne dass diese berührt werden müssten, insbesondere also ohne dass unsterile Teile des jeweiligen Geräts berührt werden müssten.

Um eine derartige berührungslose Bedienung von Geräten hinsichtlich der Erstfehlersicherheit zu verbessern und dabei gleichzeitig besonders einfach und komfortabel und flexibel zu gestalten, kann eine sequenzielle Nutzung der zur Verfügung stehenden unterschiedlichen Bedienmöglichkeiten oder Bedienkanäle, das heißt der unabhängigen Erfassungen der jeweiligen Interaktion oder Interaktionen, vorgesehen sein. Mit anderen Worten kann also beispielsweise zunächst durch eine berührungslose Interaktion, beispielsweise eine Bediengeste, eine auszuführende Funktion oder Funktionssequenz ausgewählt oder vorgegeben werden, welche dann anschließend durch Interaktion mit dem, insbesondere sterilen, Darstellungselement, insbesondere durch Berührung des Darstellungselements, bestätigt oder freigegeben und erst dann tatsächlich an das Gerät übermittelt oder von diesem ausgeführt wird.

Die Freigabe oder Bestätigung kann ebenso mittels einer anderen Auslöseeinrichtung, beispielsweise mittels eines Fußschalters erfolgen. Letzteres muss dabei vorteilhaft nicht notwendigerweise steril sein. Es kann dann also beispielsweise vorgesehen sein, zunächst mittels einer, insbesondere eine Sterilität aufrechterhaltenden, Bediengeste eine Funktion, beispielsweise eine Bewegung in eine Soll- oder Zielposition, vorzugeben. Anschließend wird diese von der Bedienperson vorgegebene Funktion durch Betätigen der Auslöseeinrichtung ausgelöst und real umgesetzt. Es kann durch die Verwendung von Methoden der augmentierten und/oder virtuellen Realität vorteilhaft beispielsweise ein virtuelles Modell (Avatar) des zu bedienenden medizinischen Geräts projiziert oder dargestellt werden. Dieses virtuelle Modell kann dann durch die Bedienperson durch entsprechende Bediengesten in die Zielposition bewegt werden. Durch dieses Vorgehen können vorteilhaft beispielsweise mögliche Konflikt- oder Kontaktpunkte bereits vor der Durchführung der realen Bewegung erkannt werden. Ebenso können unnötige reale Gerätebewegungen oder Positionskorrekturen vermieden werden, wodurch vorteilhaft eine mechanische Belastung oder Beanspruchung des Geräts minimiert wird.

Eine zweistufige oder sequentielle Bedienschrittfolge mit zunächst rein virtueller Vorgabe, anschließender Freigabe und erst dann erfolgender realer Umsetzung der Vorgabe ist auch deshalb besonders vorteilhaft, da während der Vorgabe der auszuführenden Funktion eine individuelle Freigabe von einzelnen Funktionen, Teilfunktionen oder Funktionsschritte unterbleiben kann. Dadurch können vorteilhaft eine Anzahl notwendiger Bedienhandlungen zum Erreichen eines Ergebnisses eingespart und eine Bedieneffizienz gesteigert sowie eine unnötige Einschränkung der Bedienperson vermieden werden.

Ebenso kann auch in umgekehrter Reihenfolge durch Interaktion mit dem Darstellungselement eine bestimmte Funktion, Funktionssequenz oder Gruppe von Funktionen freigegeben werden, welche dann durch, insbesondere berührungslose, Interaktion mit der Bedienoberfläche oder durch entsprechende Gesten, konkret angegeben, spezifiziert oder durchgeführt werden können. Durch diese sequenzielle Abfolge kann die Erstfehlersicherheit weiter verbessert werden. Zudem kann auf diese Art und Weise vorteilhaft vermieden werden, dass die jeweilige Person in einem Umfang oder einer Komplexität möglicher Bedienhandlungen eingeschränkt wird, da also die Bediengeste zur Erfassung durch die Erfassungseinrichtung und die Berührung des Darstellungselements zur Erfassung durch die Sensorik nicht gleichzeitig ausgeführt werden müssen.

In weiterer Ausgestaltung der vorliegenden Erfindung kann das Darstellungselement als Einweg- oder Einmalbauteil, das heißt also für eine einmalige Verwendung, vorgesehen sein. Dazu kann es beispielsweise herstellerseitig steril verpackt sein und zur Anwendung, beispielsweise in einem sterilen Arbeitsumfeld, ausgepackt werden. Auch hierdurch kann der Vorteil einer nicht durch eine zusätzliche Abdeckung, Folie oder Umhüllung behinderten Bedienbarkeit realisiert werden. Gleichzeitig kann bei dieser Lösung das Darstellungselement jedoch temperaturempfindliche Bestandteile, beispielsweise elektronische oder elektrische Schaltungen, eine magnetische Halterung, eine Aktuatorik zur Erzeugung des haptischen Signals, tatsächliche Tasten für eine besonders präzise und detaillierte Bedienbarkeit und/oder dergleichen mehr aufweisen. Vorteilhaft kann das Darstellungselement dann ebenso ein Kommunikationsmodul zur kabellosen Datenübertragung aufweisen, wodurch besonders vorteilhaft eine flexible Anordenbarkeit des Darstellungselements in der Arbeitsumgebung ermöglicht wird. Besonders vorteilhaft kann das Darstellungselement damit also flexibel so positioniert werden, dass jederzeit eine optimale Erreichbarkeit und Bedienbarkeit durch die Bedienperson ermöglicht wird. Die kabellose Datenübertragung kann beispielsweise direkt an das zu bedienende Gerät oder an ein anderes Element der Bedienvorrichtung, wie beispielsweise eine Datenverarbeitungseinrichtung zur Auswertung der erfassten Interaktionen und/oder zur Generierung eines Steuersignals für das zu bedienende Gerät in Abhängigkeit von den erfassten Interaktionen, gerichtet sein.

Bei einer zwei- oder mehrteiligen Ausgestaltung des Darstellungselements kann beispielsweise lediglich ein zur tatsächlichen Berührung durch die Bedienperson vorgesehenes Oberteil als steril verpacktes Einwegbauteil vorgesehen sein, während beispielsweise ein dauerhaft oder mehrfach verwendbares Unterteil als Empfangsstation zum Empfang der von dem Bedienoder Oberteil ausgehenden Datenübertragung dienen kann. Hierdurch kann vorteilhaft ein Material- und Kostenaufwand der Verwendung der Bedienvorrichtung minimiert werden.

In weiterer vorteilhafter Ausgestaltung der vorliegenden Erfindung kann es vorgesehen sein, dass die Interaktion mit dem Darstellungselement über ein bewegliches Bedienelement erfolgt. Das bewegliche Bedienelement kann beispielsweise ein Eingabestift (Stylus) sein, mittels welchem die jeweilige Bedienperson mit der projizierten Bedienoberfläche interagieren kann, indem beispielsweise das Darstellungselement mit dem mobilen Eingabeelement berührt und/oder letzteres auf dem Darstellungselement entlang geführt wird, wobei diese Berührung mittels der Sensorik erfasst und gegebenenfalls nachverfolgt wird. Dadurch kann vorteilhaft eine präzisere Bedienung ermöglicht werden. Es ist somit möglich, dass die Bedienperson nur in direkten Berührungskontakt mit dem mobilen Eingabeelement gerät, sodass es gegebenenfalls ausreichend sein kann, dessen Sterilität sicherzustellen. Dadurch kann die Sterilität des Arbeitsumfeldes mit besonders geringem Aufwand sichergestellt werden. Durch ein derartiges Eingabeelement können vorteilhaft bestimmte Interaktionen oder Bedienanweisungen besonders einfach und präzise vorgenommen werden, beispielsweise wenn Linien, Umrisse, Trajektorien, Verfahrwege oder dergleichen mehr angezeigt oder gezeichnet werden.

Ein erfindungsgemäßes Bedienverfahren dient zum Bedienen eines medizinischen Gerätes mittels einer Bedienvorrichtung, insbesondere mittels einer erfindungsgemäßen Bedienvorrichtung. Um eine verbesserte erstfehlersichere Bedienung des medizinischen Geräts zu ermöglichen, ist es dabei erfindungsgemäß vorgesehen, dass eine Bedienoberfläche für das medizinische Gerät reell oder virtuell auf ein Darstellungselement der Bedienvorrichtung projiziert wird. Interagiert eine Bedienperson mit dieser projizierten Bedienoberfläche, so wird diese Interaktion berührungslos erfasst. Zudem wird die Interaktion unabhängig von dem berührungslosen Erfassen der Interaktion detektiert. Von der Bedienvorrichtung wird dann in Abhängigkeit von der erfassten und detektierten Interaktion ein Steuersignal für das medizinische Gerät generiert und an das medizinische Gerät übermittelt.

In vorteilhafter Ausgestaltung der vorliegenden Erfindung ist es vorgesehen, dass mittels der Bedienvorrichtung eine Plausibilitätsprüfung jeweiliger, die Interaktion charakterisierender Ergebnisse des Erfassens - etwa mittels der Erfassungseinrichtung - und des Detektierens - etwa mittels der Sensorik - der Interaktion durchgeführt wird. Das Steuersignal für das medizinische Gerät wird nur dann an dieses übermittelt, wenn die Plausibilitätsprüfung ergibt, dass die beiden Ergebnisse zueinander plausibel sind. Je nach Ausgestaltung der Bedienvorrichtung kann durch die Plausibilitätsprüfung beispielsweise bestimmt werden, ob die erfasste Interaktion der detektierten Interaktion entspricht und/oder ob das Erfassen und das Detektieren gleichzeitig oder beispielsweise innerhalb eines vorgegebenen zeitlichen Abstandes zueinander erfolgt sind.

Die Erfassungseinrichtung und/oder die Sensorik kann dazu eingerichtet sein, eine zeitliche Abfolge mehrerer Interaktionen und/oder von Teilen einer Interaktion zu erfassen und - insbesondere hinsichtlich einer Plausibilität - auszuwerten. So kann etwa nicht nur eine Betätigung, beispielsweise ein Klicken oder Berühren, erfasst werden, sondern auch eine zuvor erfolge Annäherung, beispielsweise einer Hand, eines Finger und/oder eines Bedienobjekts, an das Darstellungselement und/oder an ein vorgegebenes Raumvolumen, welches zur Durchführung von Bedienhandlungen, insbesondere von Bediengesten, bestimmt ist. Wird eine solche der Betätigung oder Bedienhandlung vorausgehende Annäherung nicht erfasst, so kann gegebenenfalls auf eine Fehlauslösung geschlossen werden. Durch eine Berücksichtigung einer zeitlichen Abfolge können somit ein Sicherheitsgewinn und eine verbesserte Zuverlässigkeit erzielt werden.

Das erfindungsgemäße Bedienverfahren kann beispielsweise mittels der erfindungsgemäßen Bedienvorrichtung beziehungsweise unter Verwendung der im Zusammenhang mit der erfindungsgemäßen Bedienvorrichtung erläuterten Einrichtungen durchgeführt werden.

Die bisher und im Folgenden angegebenen Eigenschaften und Weiterbildungen der erfindungsgemäßen Bedienvorrichtung sowie des erfindungsgemäßen Verfahrens und die entsprechenden Vorteile sind jeweils sinngemäß wechselseitig zwischen der erfindungsgemäßen Bedienvorrichtung und dem erfindungsgemäßen Bedienverfahren übertragbar. Dies gilt auch für zur Durchführung des erfindungsgemäßen Verfahrens verwendete oder verwendbare Bauteile und Einrichtungen. Aus diesem Grund wird vorliegend auf eine jeweilige explizite Ausformulierung jedes Aspektes sowohl für die erfindungsgemäße Bedienvorrichtung als auch für das erfindungsgemäße Bedienverfahren verzichtet.

Weitere Merkmale, Einzelheiten und Vorteile der Erfindung ergeben sich aus der nachfolgenden Beschreibung bevorzugter Ausführungsbeispiele sowie anhand der Zeichnungen, welche die Erfindung erläutern. Dabei zeigen:
- FIG 1: eine schematische Perspektivansicht eines medizinischen Geräts mit einer schematisch angedeuteten Bedienvorrichtung;
- FIG 2: eine schematische Draufsicht auf zwei unterschiedliche Darstellungselemente;
- FIG 3: eine schematische und ausschnittweise Perspektivansicht eines weiteren medizinischen Geräts mit einem daran angeordneten Darstellungselement;
- FIG 4: eine schematische Darstellung eines weiteren medizinischen Geräts mit einem daran angeordneten Darstellungselement in zwei unterschiedlichen Stellungen;
- FIG 5: eine schematische und ausschnittsweise Darstellung eines weiteren medizinischen Geräts mit einem daran angeordneten Darstellungselement;
- FIG 6: eine schematische Darstellung verschiedener Bedieneinrichtungen zur Realisierung einer mehrkanaligen, erstfehlersicheren Bedienung;
- FIG 7: eine schematische Perspektivdarstellung eines medizinischen Geräts mit einem virtuell visualisierten Strahlkegel; und
- FIG 8 bis FIG 11: schematische Perspektivdarstellungen des medizinischen Geräts aus FIG 7 mit schematischen Veranschaulichungen unterschiedlicher berührungsloser Interaktionsmöglichkeiten.

In den FIG sind gleiche, funktionsgleiche oder einander entsprechende Elemente jeweils mit den gleichen Bezugszeichen gekennzeichnet.

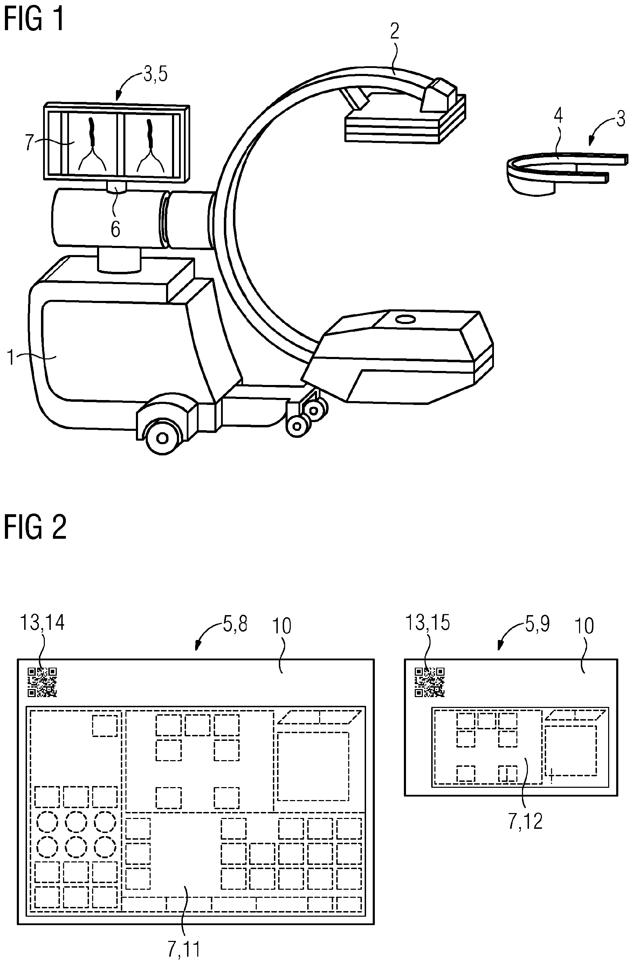

FIG 1 zeigt eine schematische Darstellung eines medizinischen Geräts 1, bei dem es sich vorliegend um ein mobiles Röntgengerät mit einem C-Bogen 2 handelt.

Medizinische Geräte verfügen heutzutage oftmals über vielfältige Funktionen, Funktionalitäten, mechanische Freiheitsgrade und entsprechend zahlreiche und komplexe Bedienelemente. Dabei ist es bisher durchaus üblich, dass eine Bedienung dieser Geräte und deren vielfältiger Funktionen über Bedienelemente erfolgt, welche an unterschiedlichen Stellen des jeweiligen Geräts angeordnet sind. Das bedeutet, dass nachteilig einer jeweiligen Bedienperson, welche beispielsweise unmittelbar neben dem C-Bogen 2 steht und arbeitet, nicht alle Funktionen in Griffreichweite zur Verfügung stehen. Dabei ist auch zu beachten, dass Geräte - wie etwa das vorliegend dargestellte mobile Röntgengerät - in Operationsräumen oder bei intraoperativen Anwendungen, insbesondere also in sterilen Umgebungen eingesetzt werden. Dabei ergibt sich das Problem, dass die Bedienperson oftmals nicht alle möglichen Funktionen bedienen kann, ohne zumindest teilweise den sterilen Bereich zu verlassen. Auch eine Verdeckung von Bedienelementen, Anzeigen oder Monitoren, beispielsweise durch Teile des medizinischen Geräts 1 oder weitere umstehende Personen kann zumindest für bestimmte Funktionen oder Anwendungen, beispielsweise eine Justierung einer Blende oder dergleichen, problematisch sein. Insgesamt ergibt sich also das Problem, in einem derartigen anspruchsvollen Umfeld eine Bedienmöglichkeit für komplexe Geräte zu schaffen, welche eine ergonomische Bedienung der komplexen Funktionen erlaubt, den Anforderungen an eine sterile Bedienbarkeit gerecht wird und zudem eine Erstfehlersicherheit, beispielsweise gemäß der Norm IEC6060-1, bietet.

Um diesen Problemen zu begegnen, ist es vorliegend vorgesehen, dass das medizinische Gerät 1 mittels einer hier schematisch dargestellten Bedienvorrichtung 3 bedient wird. Die Bedienvorrichtung 3 umfasst vorliegend eine Projektionseinrichtung in Form einer Datenbrille 4 sowie ein Darstellungselement 5, welches vorliegend an dem medizinischen Gerät 1 angeordnet ist. Darüber hinaus kann die Bedienvorrichtung 3 auch noch weitere, hier nicht explizit dargestellte Elemente umfassen. Das Darstellungselement 5 ist hier über eine Verbindung 6, beispielsweise eine Halterung, Aufnahme oder Abstützung, mit dem medizinischen Gerät 1 verbunden.

Die Datenbrille 4 kann beispielsweise eine Augmented-Reality-Brille sein und dazu beispielsweise über eine oder mehrere 3D- oder Tiefen- und RGB-Kameras zur Umgebungserfassung, Projektionselemente zur stereoskopischen Bilddarstellung, einen Bewegungssensor (Headtracking) und eine Datenverarbeitungseinheit umfassen. Zusätzlich kann beispielsweise ein Lautsprecher und/oder ein Mikrofon als Teil der Datenbrille 4 oder der Bedienvorrichtung 3 vorgesehen sein, wodurch beispielsweise eine unterstützende Bedienung mittels Sprachsteuerung oder Sprachbefehlen ermöglicht werden kann. Mittels der Datenbrille 4 kann also die jeweilige Umgebung, einschließlich des medizinischen Geräts 1 und des Darstellungselements 5, erfasst werden. Ebenfalls können Bediengesten der Bedienperson erfasst werden, welche beispielsweise im freien Raum oder insbesondere unmittelbar an oder vor dem Darstellungselement 5 ausgeführt werden. Mittels der Datenbrille 4 kann eine Zuordnung zwischen realen räumlich lokalisierten Objekten, wie dem medizinischen Gerät 1 und dem Darstellungselement 5 und einer virtuellen Umgebung oder virtuellen Objekten oder Repräsentation vorgenommen werden. Somit wird also der Bedienperson durch die Datenbrille 4 eine Verwendung von Funktionen und Bedienhandlungen der virtuellen, augmentierten oder gemischten Realität ermöglicht. Die Datenverarbeitungseinheit kann dabei auch separat von der Datenbrille angeordnet sein, beispielsweise in Form eines stationären Rechnersystems.

Die Datenbrille 4 bildet also, gegebenenfalls beispielsweise in Verbindung mit einer hier nicht dargestellten weiteren Kamera, eine Erfassungseinrichtung zum berührungslosen Erfassen einer Bedienhandlung der Bedienperson. Vorliegend ist es dabei vorgesehen, dass auf das Darstellungselement 5 eine Bedienoberfläche virtuell projiziert wird, sodass die Bedienoberfläche 7 also für die die Datenbrille 4 tragende Bedienperson auf oder an dem Darstellungselement 5 erscheint. Aus Sicht der Bedienperson erscheint die Bedienoberfläche 7 also ortsfest relativ zu dem Darstellungselement 5. Dabei erfolgt die Darstellung der Bedienoberfläche 7 jeweils perspektivisch korrekt an eine jeweilige Position und einen jeweiligen Blickwinkel der Bedienperson relativ zu dem Darstellungselement 5 angepasst.

Vorliegend ist es dabei vorgesehen, dass das Darstellungselement 5 beispielsweise zweiteilig aus einem Oberteil und einem Unterteil gebildet ist. Das Unterteil ist auf einer von der Bedienperson abgewandten Seite mit dem medizinischen Gerät 1 verbunden, während das Oberteil eine der Bedienperson zugewandte Darstellungsfläche 10 (vergleiche FIG 2) umfasst, welche der - gegebenenfalls virtuellen - Projektion der Bedienoberfläche 7 beziehungsweise als entsprechender Hintergrund für die Bedienoberfläche 7 dient. Bevorzugt wird das Oberteil des Darstellungselements 5 durch ein beschädigungsfrei dampfsterilisierbares, beispielsweise plattenförmiges, Element gebildet. Dieses kann dann vorteilhaft von dem Unterteil und somit auch von dem medizinischen Gerät 1 gelöst und einzeln sicher sterilisiert werden. Das Unterteil umfasst dabei eine Sensorik, mittels welcher eine Berührung des Darstellungselements 5, beispielsweise ein Druck darauf und/oder eine resultierende Bewegung des Darstellungselements 5 beziehungsweise des Oberteils detektierbar ist. Dazu kann die Sensorik beispielsweise einen Drucksensor umfassen, welcher beispielsweise in oder an der Verbindung 6 und/oder zwischen dem Oberteil und dem Unterteil, insbesondere an einem jeweiligen Berührungspunkt zwischen dem Unterteil und dem Oberteil, angeordnet sein kann.

Die Bedienoberfläche 7 kann beispielsweise mehrere einzelne Bedienelemente umfassen oder darstellen, welche mit entsprechenden Funktionen des medizinischen Geräts 1 korrespondieren. Zusätzlich oder alternativ können als Teil der Bedienoberfläche auch Daten, wie beispielsweise medizinische Bilddaten, Röntgenbilder, Endoskopiedaten und dergleichen, dargestellt werden.

Der Bedienperson wird durch die Bedienvorrichtung 3 über die Einblendung der Bedienoberfläche, beispielsweise mittels der stereoskopischen Anzeigevorrichtung der Datenbrille 4, vor seinen Augen die nicht real physisch an dem Darstellungselement 5 vorhandene Benutzeroberfläche 7 angezeigt, mit welcher sie aber dennoch interagieren kann. Die Interaktion, also beispielsweise eine Bedienhandlung, insbesondere eine Bediengeste, wird dabei sowohl von der Erfassungseinrichtung, also beispielsweise der Gestenerfassung der Datenbrille 4, als auch von der an dem Darstellungselement vorgesehenen Sensorik unabhängig und basierend auf unterschiedlichen Funktionsprinzipien erfasst. Nimmt die Bedienperson also eine derartige Interaktion oder Bedienhandlung zum Bedienen oder Steuern des medizinischen Geräts 1 vor, indem sie eine entsprechende Geste macht, so wird dies von der Bedienvorrichtung 3 erfasst und in ein entsprechend von der Art der erfassten Interaktion oder Bedienhandlung abhängiges Steuersignal für das medizinische Gerät 1 umgewandelt, welches dann an das medizinische Gerät übermittelt wird. Dabei ist es vorgesehen, dass das medizinische Gerät 1 nur gemäß der erfassten Interaktion angesteuert wird, wenn die mittels der Erfassungseinrichtung erfasste Interaktion und die mittels der Sensorik erfasste Interaktion einander entsprechen oder zueinander plausibel sind. Hierdurch wird vorteilhaft eine versehentliche Bedienung vermieden und somit eine Erstfehlersicherheit erreicht.

Die Bedienhandlung oder Interaktion kann beispielsweise eine Berührung eines bestimmten, einer bestimmten Funktion des medizinischen Geräts 1 zugeordneten Teilbereichs des Darstellungselements 5 sein. Somit ist also beispielsweise eine Bedienung virtuell dargestellter Bedienknöpfe, Tasten, Schieberegler und dergleichen mehr möglich. Um insbesondere komplexe Bedienhandlungen beziehungsweise Gesten zur Bedienung des medizinischen Geräts 1 verwenden zu können, kann es auch vorgesehen sein, dass eine Ausführung der entsprechenden, durch eine oder mehrere Gesten vorgegebenen Funktion oder Funktionen beziehungsweise Anweisungen erst auf eine Freigabe hin erfolgt, welche beispielsweise durch physische Berührung des Darstellungselements 5 und/oder eines weiteren Bedien-, Freigabe- oder Auslöseelementes, beispielsweise eines Fußschalters, erfolgen kann.

FIG 2 zeigt eine schematische Draufsicht auf zwei unterschiedliche Typen von Darstellungselementen 5. Konkret sind ein großes Darstellungselement 8 und ein kleines Darstellungselement 9 gezeigt, welche sich vorliegend insbesondere hinsichtlich ihrer Größe unterscheiden. Dementsprechend kann auf dem großen Darstellungselement 8 eine erweiterte Bedienoberfläche 11 mit einer Vielzahl von einzelnen Bedienelementen oder Funktionsbereichen dargestellt werden, welche offensichtlich nicht sinnvoll auf dem kleinen Darstellungselement 9 dargestellt werden können. Auf dem kleinen Darstellungselement 9 ist deshalb vorliegend eine beschränkte Bedienoberfläche 12 mit einer alternativen Auswahl und Anordnung von Bedienelementen oder Funktionsbereichen dargestellt. Die beschränkte Bedienoberfläche kann beispielsweise kleiner skaliert sein als die erweiterte Bedienoberfläche 11 und/oder gegenüber dieser vereinfacht beziehungsweise funktionsreduziert sein. Die Bedienoberflächen 11, 12 sind also jeweils an das große Darstellungselement 8 beziehungsweise das kleine Darstellungselement 9 angepasst.

Um diese Anpassung besonders zuverlässig und automatisiert durchführen zu können, weisen die Darstellungselemente 8, 9 jeweils eine maschinenlesbare Kennzeichnung 13 auf, welche beispielsweise mittels der Erfassungseinrichtung der Bedienvorrichtung 3 erfasst, ausgelesen und ausgewertet werden kann. Vorliegend handelt es sich bei der Kennzeichnung 13 um einen QR-Code, welcher einen jeweiligen individuellen Typ, hier also beispielsweise eine Größe, des jeweiligen Darstellungselements 8, 9 kodiert. Konkret umfasst hier das große Darstellungselement 8 eine Kennzeichnung 14, welche zumindest eine Eigenschaft des großen Darstellungselements 8 als Dateninhalt aufweist. Entsprechend umfasst das kleine Darstellungselement 9 vorliegend eine Kennzeichnung 15, welche zumindest eine Eigenschaft des kleinen Darstellungselements 9 als Dateninhalt aufweist. Auch die übrigen, beispielsweise in den anderen FIG dargestellten, Darstellungselemente 5 können derartige individuelle maschinenlesbare Kennzeichnungen 13 aufweisen.

Durch die Kennzeichnungen 13 kann ein eindeutiges Erkennen und Identifizieren des jeweiligen Darstellungselements 5 beziehungsweise 8, 9 auch unter schwierigen Bedingungen ermöglicht oder erleichtert werden, um eine zuverlässige Projektion oder Darstellung der projizierten Bedienoberfläche 7, 11, 12 zu erreichen. Dabei kann mittels der Kennzeichnung 13 beispielsweise auch eine räumliche, zum Beispiel horizontale oder vertikale, Ausrichtung des jeweiligen Darstellungselements 5 angegeben werden. Vorteilhaft können derartige Kennzeichnungen 13 auch dafür genutzt werden, mehr als ein Darstellungselement 5 gleichzeitig zu verwenden und dennoch eine eindeutige Zuordnung und Identifizierung zu ermöglichen. So kann beispielsweise auch bei einer Perspektivänderung und/oder einer Verdeckung eines der Darstellungselemente 5 sichergestellt werden, dass stets auf einem bestimmten Darstellungselement 5 die jeweils vorgesehene Bedienoberfläche 7, 11, 12 dargestellt wird.

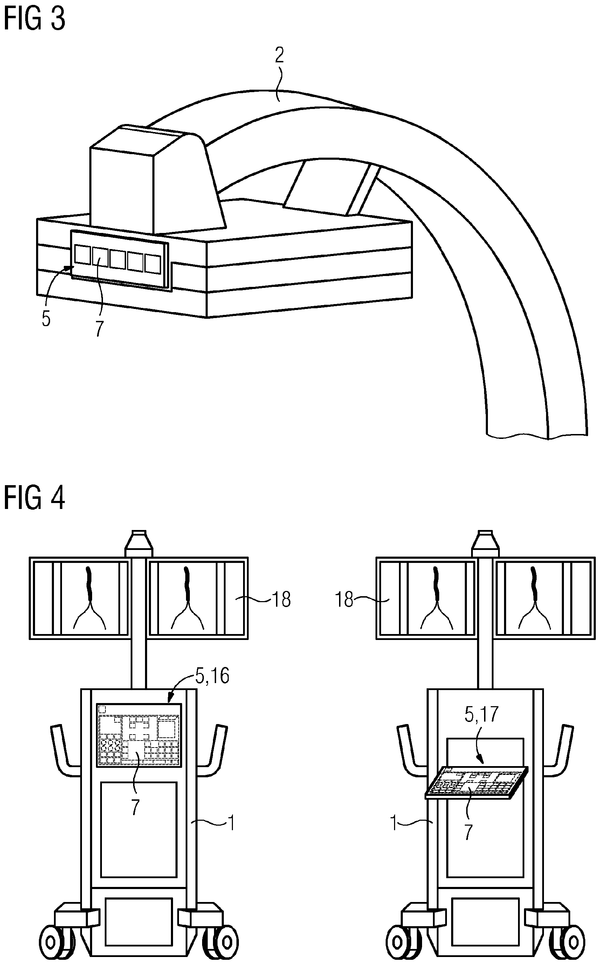

FIG 3 zeigt eine schematische und ausschnittweise Darstellung eines C-Bogens 2 mit einem Flachdetektor, an welchem ein Darstellungselement 5 angeordnet ist. Auch hier ist an die Form und Größe des Darstellungselements 5 angepasst eine Bedienoberfläche 7 schematisch dargestellt. Hierdurch wird also ein weiteres Anwendungsbeispiel illustriert.

FIG 4 zeigt eine weitere schematische Darstellung eines medizinischen Geräts 1, bei dem es sich vorliegend um einen Monitortrolley handelt. Vorliegend ist an dem Monitortrolley ein Darstellungselement 5 angeordnet, welches in der linken Teildarstellung in einer ersten Stellung 16 und in der rechten Teildarstellung in einer zweiten Stellung 17 angeordnet ist. Das Darstellungselement 5 kann also beispielsweise zwischen diesen beiden Stellungen 16, 17 klappbar oder verschwenkbar sein. Hierdurch kann vorteilhaft die Stellung, Anordnung oder Ausrichtung des Darstellungselements 5 situations- beziehungsweise bedarfsgerecht angepasst oder eingestellt werden. Vorliegend umfasst der Monitortrolley wenigstens einen herkömmlichen Bildschirm 18, welcher beispielsweise zur Darstellung medizinischer Daten oder Bilder nutzbar ist.

Da die jeweils verfügbare Monitorfläche des Monitors 18 stets begrenzt ist, kann aufgrund der besonderen Flexibilität der Bedienvorrichtung 3 vorteilhaft auch das Darstellungselement 5 zur Darstellung derartigen Bildmaterials genutzt werden. Es kann also beispielsweise bedarfsgerecht zwischen einer solchen Darstellung von medizinischen oder sonstigen Bilddaten und der Darstellung der Bedienoberfläche 7 gewechselt werden. Besonders vorteilhaft kann bei einer rein virtuellen Projektion oder Darstellung auf dem Darstellungselement 5 für unterschiedliche Personen, welche jeweils eine eigene Datenbrille 4 verwenden, eine unterschiedliche, jeweils individuell bedarfsgerecht angepasste Darstellung erfolgen. So kann beispielsweise das Darstellungselement 5 einer ersten Bedienperson zur Darstellung beziehungsweise als Projektionsfläche oder Projektionsplatte für eine Bedienoberfläche 7 dienen, während es gleichzeitig einer zweiten Bedienperson beispielsweise zur virtuellen Darstellung eines Röntgenbildes oder dergleichen dienen kann. Hierdurch wird also besonders vorteilhaft eine Mehrfachnutzung des Darstellungselements 5 ermöglicht.

Vorteilhaft kann so das Darstellungselement 5 beispielsweise auch unterstützend für weiteres OP-Personal als Anzeigefläche dienen, wenn beispielsweise für dieses Personal eine jeweilige Sicht auf den dedizierten Bildschirm 18 eingeschränkt ist.

In entsprechender Art und Weise kann das Darstellungselement 5 beziehungsweise ein weiteres Darstellungselement 5 oder eine Mehrzahl weiterer Darstellungselemente 5 beispielsweise auch an einem ausziehbare Arm oder einem Stativ an dem medizinischen Gerät 1, an dem C-Bogen 2 und/oder beispielsweise an einer Wand oder einer Decke eines jeweiligen Raumes angeordnet beziehungsweise gehalten sein.

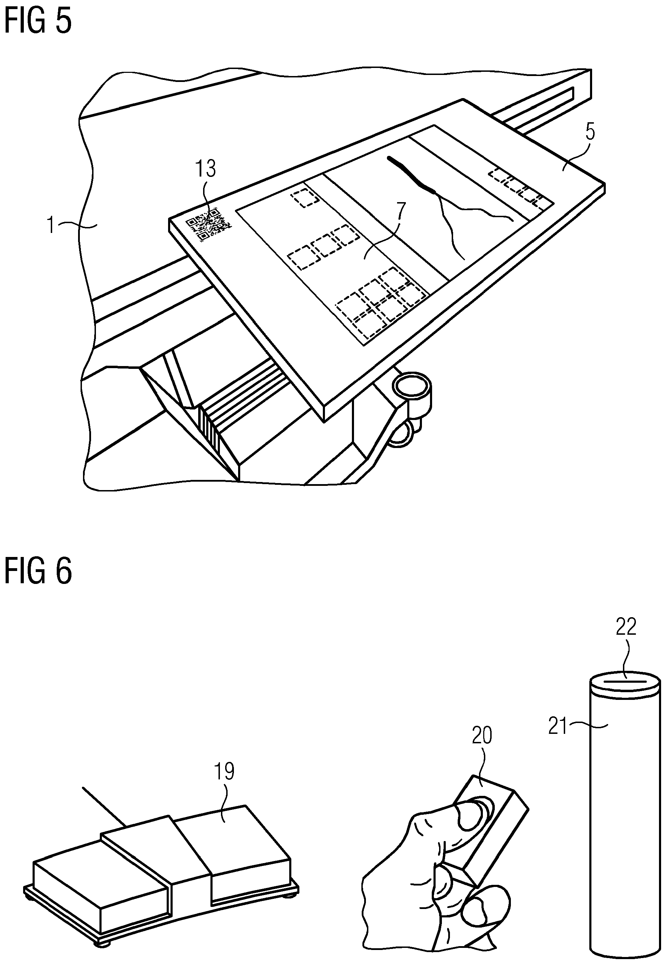

FIG 5 zeigt in einer schematischen und ausschnittweisen Darstellung ein weiteres Beispiel eines medizinischen Geräts 1 mit einem daran angeordneten Darstellungselement 5. In dem hier gezeigten Beispiel handelt es sich bei dem medizinischen Gerät 1 um eine Patientenliege oder einen Operationstisch.

FIG 6 zeigt in einer schematischen Übersichtsdarstellung drei beispielhafte Realisierung zusätzlicher Bedieneinrichtungen. Mittels solcher zusätzlicher Bedieneinrichtungen kann beispielsweise in besonders sicherheitskritischen Situationen beziehungsweise für besonders sicherheitskritische Funktionen oder Funktionsauslösungen eine separate Freigabe oder Bestätigung durch die Bedienperson vorgenommen werden, sodass also eine zwei- oder mehrkanalige Bedienung ermöglicht beziehungsweise sichergestellt ist. Eine jeweilige Betätigung dieser zusätzlichen Bedieneinrichtungen kann ein Freigabe- oder Zustimmsignal erzeugen, welches an die Bedienvorrichtung 3 oder eine Datenverarbeitungseinrichtung der Bedienvorrichtung 3 übermittelt wird.

Es kann beispielsweise vorgesehen sein, dass eine Funktion des medizinischen Geräts 1 nur dann ausgelöst wird, wenn gleichzeitig oder parallel mit der entsprechenden Bedienhandlung ein derartiges Zustimmsignal mittels einer der zusätzlichen Bedieneinrichtungen erzeugt beziehungsweise von der Bedieneinrichtung 3 empfangen wird. Es kann auch ein vorgegebener zeitlicher Abstand definiert sein, welcher maximal zwischen der jeweiligen Bedienhandlung und dem zugehörigen Zustimmsignal liegen, das heißt verstreichen darf. Wird innerhalb dieses vorgegebenen Zeitraums in Bezug auf einen Zeitpunkt der jeweiligen Bedienhandlung kein entsprechendes Zustimmsignal empfangen, so wird das der Bedienhandlung entsprechende Steuersignal nicht an das medizinische Gerät 1 übermittelt, die entsprechende Funktion also nicht ausgelöst.

Vorliegend sind beispielhaft ein Fußpedal 19, ein händisch bedienbarer Taster 20 sowie ein Zylinder 21 mit einer Freigabetaste 22 dargestellt. Der Taster 20 kann beispielsweise eine Totmanntaste umfassen, welche beispielsweise zur Bedienung oder Ausführung sicherheitskritischer Funktionen permanent in einem betätigten oder gedrückten Zustand gehalten werden muss. Der Taster 20 und der Zylinder 21 können als Handsender ausgebildet sein, wobei das jeweilige Freigabe- oder Zustimmsignal beispielsweise kabellos gesendet werden kann.

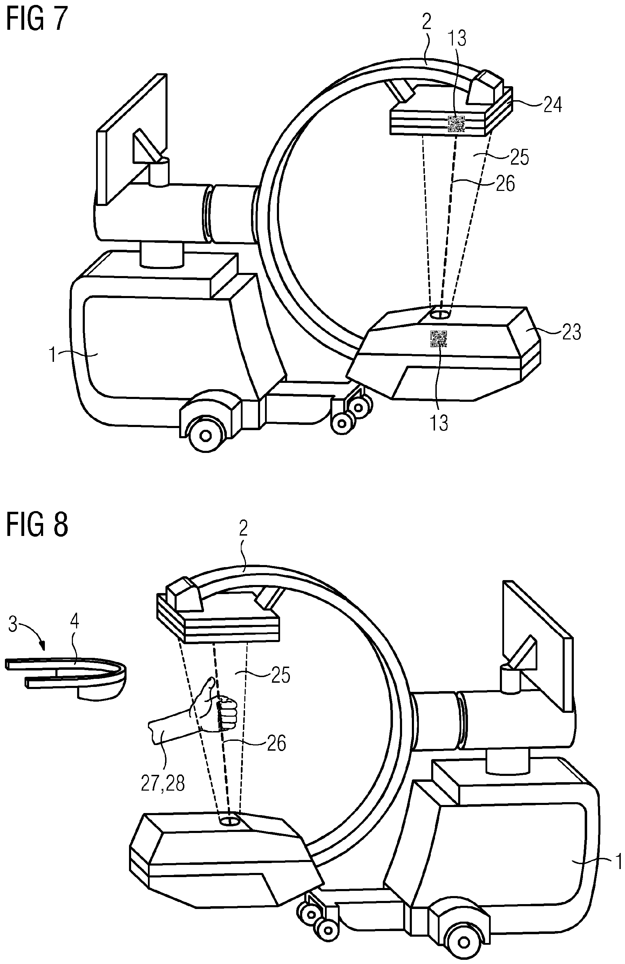

FIG 7 zeigt eine schematische Perspektivdarstellung eines medizinischen Geräts 1, welches im Wesentlichen dem bereits in FIG 1 gezeigten Gerät entspricht. Es handelt sich also auch bei dem in FIG 7 dargestellten medizinischen Gerät um ein mobiles Röntgengerät mit einem C-Bogen 2, an welchem vorliegend eine Strahlquelle 23 und ein dieser gegenüberliegend angeordneter Detektor 24 gehalten sind. Sowohl die Strahlquelle 23 als auch der Detektor 24 weisen vorliegend jeweils zumindest eine maschinenlesbare Kennzeichnung 13 auf, welche mittels der Erfassungseinrichtung lesbar ist. Mittels der Kennzeichnungen 13 der Strahlquelle 23 und des Detektors 24 kann besonders einfach und zuverlässig eine eindeutige Identifizierung und Lokalisierung des C-Bogens 2 im Raum mittels der Bedienvorrichtung 3 erreicht werden. Dies kann insbesondere bei einer teilweisen Verdeckung des C-Bogens 2 und/oder ungünstiger Ausleuchtung die Identifizierung und Lokalisierung erleichtern und somit verlässlicher gestalten.

Alternativ oder unterstützend kann die Lokalisierung und/oder Identifizierung des C-Bogens 2 beziehungsweise des medizinischen Geräts 1 mittels beispielsweise mittels einer Bild- oder Mustererkennung, eines Oberflächenscans (surface scanning) - bedarfsweise zuzüglich eines Abgleichs mit einem Volumenmodell - oder dergleichen erfolgen.

Die Kennzeichnungen 13 können an einem bekannten vorgegebenen Punkt der Strahlquelle 23 und des Detektors 24 angeordnet sein, wodurch eine besonders präzise Einblendung oder Überlagerung virtueller Elemente erleichtert wird. Dadurch kann vorteilhaft sichergestellt werden, dass physisch reale und virtuelle Elemente aus Sicht der Bedienperson konsistent relativ zueinander angeordnet sind.

Vorliegend ist beispielhaft ein Strahlkegel 25 angedeutet welcher als virtuelles Element dargestellt wird, nachdem die räumliche Lage des C-Bogen 2 relativ zu der von der Bedienperson getragenen Datenbrille 4 zumindest initial ermittelt worden ist. Der Strahlkegel 25 ist real unsichtbar, da es sich beispielsweise um einen Strahlengang von von der Strahlquelle 23 emittierter Röntgenstrahlung handeln kann. Der Strahlkegel 25 wird vorliegend in Form eines perspektivisch korrekt stereoskopisch virtuell projizierten Pyramidenstumpfes zwischen der Strahlquelle 23 und dem Detektor 24 dargestellt. Die tatsächliche oder reale Form des Strahlkegels 25 kann von einer verwendeten, beispielsweise runden oder rechteckigen, Blende abhängen, sodass hier der Begriff "Strahlkegel" breit ausgelegt werden und nicht auf geometrische Kegelformen beschränkt sein soll.

Zusätzlich ist vorliegend ein Zentralstrahl 26 des Strahlkegels 25 dargestellt. Der Zentralstrahl 26 kann beispielsweise als Linie visualisiert werden und als Referenzobjekt für Bediengesten dienen. Bereits eine nicht interaktive Darstellung des Strahlkegel 25 und/oder des Zentralstrahls 26 kann für eine jeweilige Bedienperson einen Mehrwert bieten, da unsichtbare Strahlung zumindest virtuell sichtbar gemacht und dadurch besonders anschaulich wird, insbesondere in ihrer räumlichen Relation bezüglich eines Untersuchungsobjekts. Hierdurch kann beispielsweise eine korrekte Ausrichtung des C-Bogens 2 relativ zu dem Untersuchungsobjekt erleichtert werden, wobei vorteilhaft heute alternativ eingesetzte Positionierlaser eingespart werden können. Dabei ist auch zu beachten, dass ein Positionierlaser das Untersuchungsobjekt gegebenenfalls nicht durchdringen kann, sodass der Verlauf des Zentralstrahls 26 gegebenenfalls nur auf einer Seite des Untersuchungsobjekts erkennbar ist. Im Gegensatz dazu ist die virtuelle Darstellung des Strahlkegel 25 und des Zentralstrahls 26 vorteilhaft nicht durch derartige physische Gegebenheiten beschränkt.

Besonders bevorzugt kann der Strahlkegel 25 um weitere Daten angereichert werden, und beispielsweise in Abhängigkeit von einer Strahlungsintensität und/oder abhängig von einer räumlichen Verteilung einer Direkt- und einer Streustrahlung mit einem entsprechenden farblichen Verlauf oder dergleichen dargestellt werden. Dadurch kann vorteilhaft eine unnötige Strahlungsbelastung des Untersuchungsobjekts minimiert oder vermieden werden. Der Strahlkegel 25 kann beispielsweise in Abhängigkeit von einer vorab eingestellten zu verwendenden Strahlendosis und/oder in Abhängigkeit von einem vorab eingestellten zu verwendenden Spektrum, beispielsweise ebenfalls durch eine entsprechende Einfärbung, angepasst dargestellt werden. Durch derartige besonders leicht erkennbare Anpassung der Darstellung des Strahlkegels 25 kann zuverlässig eine entsprechende Fehleinstellung vermieden werden.

Die Überlagerung oder Einblendung ist dabei nicht auf den Strahlkegel 25 beschränkt. Zum Beispiel können auch dem C-Bogen 2 und/oder anderen Teilen des medizinischen Geräts 1 Daten oder Visualisierungen überlagert werden. Dadurch kann beispielsweise besonders anschaulich und schnell auch mit einem peripheren Sehen wahrnehmbar eine Eigenschaft, wie beispielsweise eine jeweils aktuelle Temperatur eines Strahlungserzeugers, visualisiert und vermittelt werden.

FIG 8 bis 11 zeigen jeweils eine schematische Perspektivdarstellung des medizinischen Geräts 1 aus FIG 7, wobei unterschiedliche berührungslose Interaktionsmöglichkeiten zu dessen Bedienung mittels einer jeweils angedeuteten Hand 27 der Bedienperson veranschaulicht sind.

In FIG 8 ist eine erste Geste 28 angedeutet, durch welche mittels eines Greifens und Ziehens des virtuell dargestellten Zentralstrahls 26 der C-Bogen 2 bewegt und ausgerichtet werden kann. Hierbei kann auch eine Rotation der Hand 27 verwendet werden, sodass eine Bewegung des C-Bogens 2 gemäß aller seiner angularen, orbitalen und translatorischen Freiheitsgrade ermöglicht wird. Die entsprechenden Gesten können beispielsweise mittels jeweiliger motorischer Antriebe des medizinischen Geräts 1 real umgesetzt werden. Hier muss also die Bedienperson das medizinische Gerät 1 zu dessen Bedienung nicht berühren.

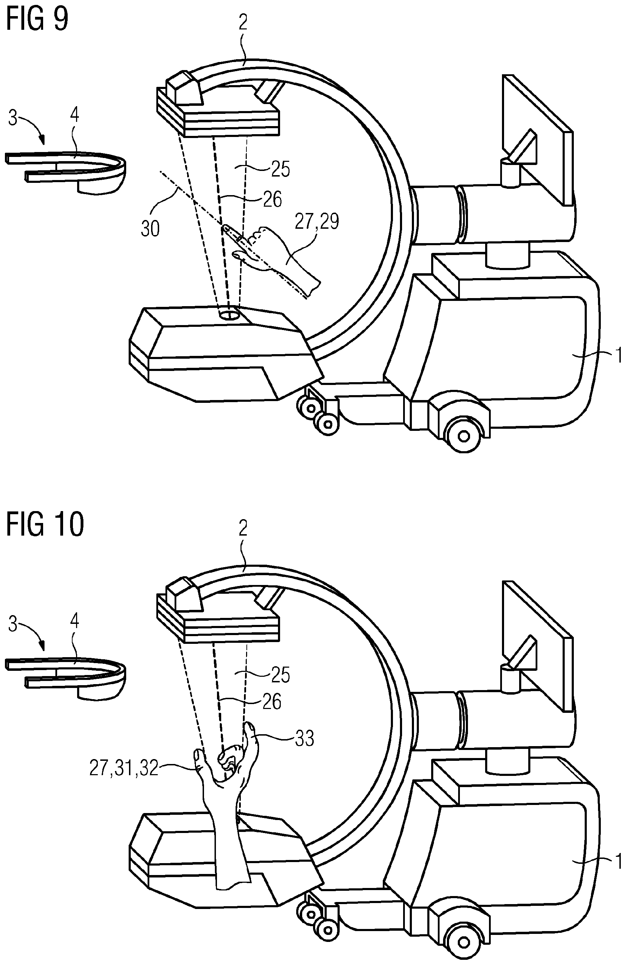

In FIG 9 ist eine zweite Geste 29 angedeutet, mittels welcher die Bedienperson durch Zeigen in eine Zeigerichtung 30 eine Neuausrichtung des C-Bogens 2 vorgeben kann. Dabei kann durch Vorgeben oder Anzeigen der Zeigerichtung 30 bewirkt werden, dass der C-Bogen 2 automatisiert derart verfahren wird, dass der Zentralstrahl 26 mit der Zeigerichtung 30 zusammenfällt. Durch die zweite Geste 29 kann beispielsweise auch auf eine zu untersuchende Untersuchungsregion (ROI, region of interest) gedeutet werden, welche dann durch Verstellen des C-Bogens 2 in Überdeckung mit dem Strahlkegel 25 gebracht wird. Mittels der zweiten Geste 29 kann ebenso ein neuer Vektor für den Zentralstrahl 26 beziehungsweise eine neue Interessenoder Zielregion vorgegeben werden. Diese kann dann in einer Liste oder Datenbank gespeichert und für zukünftige Untersuchungen abgerufen werden. So können beispielsweise besonders einfach auch komplexe Bewegungen oder Abfolgen unterschiedlicher Einzeluntersuchungen mit unterschiedlichen Durchleuchtungsrichtungen präzise und anschaulich vorgegeben werden, wobei dies gegebenenfalls auch in Abwesenheit des Untersuchungsobjekts und damit ohne Bewegung Einschränkung durchgeführt werden kann.

Die Gesten 28, 29 stellen Beispiele von Gesten dar, welche besonders anschaulich durch Verwendung des Zylinders 21 unterstützt oder erleichtert werden können. So kann beispielsweise der Zylinder 21 seinerseits eine Sensorik aufweisen mittels welcher seine genaue räumliche Ausrichtung erfasst und an die Bedienvorrichtung 3 übermittelt werden kann. Zusätzlich oder alternativ kann der Zylinder 21 gegebenenfalls besonders einfach und zuverlässig mittels der Erfassungseinrichtung der Bedienvorrichtung 3 erfasst und nachverfolgt werden. Der Bedienperson bietet der Zylinder 21 dabei eine physisch greifbare Repräsentation beispielsweise des Zentralstrahls 26, wodurch eine präzise Ausrichtung erleichtert wird. Dabei kann also eine Längsachse des Zylinders 21 als Repräsentation oder Referenzobjekt beziehungsweise Referenzrichtung für den Zentralstrahl 26 verwendet werden. Ebenso kann die Längsachse des Zylinders 21 auch zur Vorgabe der Zeigerichtung 30 verwendet beziehungsweise als Zeigerichtung 30 aufgefasst oder interpretiert werden. Besonders bevorzugt kann der Zylinder 21 beispielsweise eine Aktuatorik zur Erzeugung eines haptischen Signals aufweisen. Hierdurch wird ebenfalls eine besonders präzise Bedienung oder Steuerung des medizinischen Geräts 1 erleichtert. Beispielsweise kann durch ein entsprechendes haptisches Signal oder eine entsprechende haptische Rückmeldung, beispielsweise eine Vibration, eine erkannte Bediengeste von Seiten der Bedienvorrichtung 3 bestätigt und/oder beispielsweise ein Erreichen einer bestimmten Position oder Winkelstellung durch ein derartiges haptisches Signal signalisiert werden.

In FIG 10 ist eine dritte Geste 31 angedeutet, bei welcher beispielsweise ein erster Finger 30 und ein zweiter Finger 33 aufeinander zu oder voneinander weg bewegt werden können, um eine Breite oder Weite des Strahlkegels 25, das heißt effektiv also eine Einstellung einer Strahlenblende der Strahlquelle 23, anzupassen oder einzustellen.

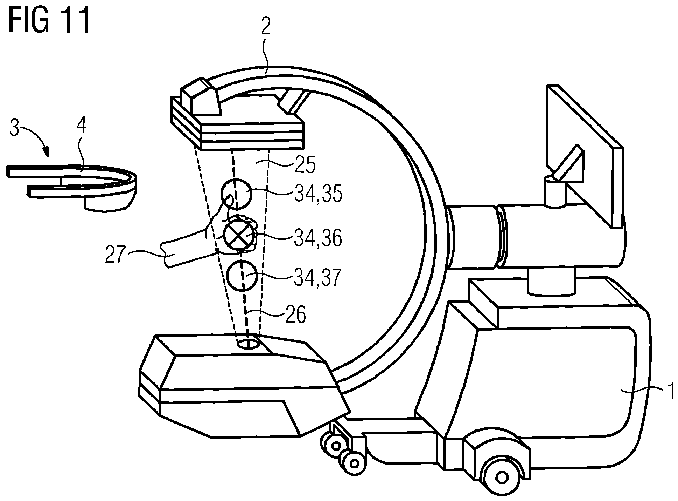

In FIG 11 ist schematisch eine Alternative oder Erweiterung der ersten Geste 28 angedeutet. Dabei ist es vorgesehen, dass rein virtuell zusätzliche Hilfsbedienelemente 34 eingeblendet werden. Beispielsweise kann die Bedienperson durch eine entsprechende Bediengeste, beispielsweise einen virtuellen Druck auf eines der Hilfsbedienelemente 34, bestimmte Bewegungsachsen des C-Bogens 2 sperren oder eine spezifische Bewegungsart auswählen, welche dann in einer nachfolgenden Interaktion, beispielsweise durch eine Ziehgeste, in ihrem Umfang spezifiziert werden kann.

Beispielsweise kann es möglich sein, exakt und ausschließlich einen Vertikalhub des C-Bogens 2 zu steuern, indem die entsprechende Bediengeste ausgehend von der virtuellen Position eines ersten Hilfsbedienelement 35 gestartet wird. Entsprechend kann beispielsweise eine an der virtuellen Position eines zweiten Hilfsbedienelement 36 aus vorgenommene oder gestartete Bediengeste dazu dienen, ausschließlich eine isozentrische orbitale Rotation des C-Bogens 2 oder beispielsweise eine lineare Seitwärtsbewegung des gesamten medizinischen Geräts 1 entlang eines Operationstischen zu veranlassen. Ein drittes Hilfsbedienelement 37 kann beispielsweise betätigt werden oder als Ausgangspunkt für eine Bediengeste dienen, mittels welcher eine angulare Rotation des C-Bogens 2 veranlasst beziehungsweise vorgenommen wird. Durch derartige Hilfsbedienelemente 34 kann also beispielsweise besonders vorteilhaft eine Einschränkung von Freiheitsgraden des C-Bogens 2 beziehungsweise des medizinischen Geräts 1 vorgenommen oder eingestellt werden, wodurch eine besonders präzise, zuverlässige und sichere Bedienung und Steuerung sowie eine besonders verlässliche Erkennung oder Interpretation der jeweiligen Bediengesten erleichtert wird.

Insgesamt zeigen die beschriebenen Beispiele, wie mittels einer neuartigen Mensch-Maschine-Schnittstelle basierend auf virtueller Projektion beziehungsweise augmentierter Realität eine Bedienbarkeit und ein klinischer Workflow vereinfacht und neuartige Darstellungsmöglichkeiten genutzt werden können und dabei die Sterilität der Bedienelement sichergestellt werden kann.

## Patentansprüche

1. Bedienvorrichtung (3) für ein medizinisches Gerät (1), mit einer Projektionseinrichtung (4), welche zur reellen und/oder virtuellen Projektion einer Bedienoberfläche (7, 11, 12) auf ein Darstellungselement (5, 8, 9) eingerichtet ist, und mit einer Erfassungseinrichtung zum berührungslosen Erfassen einer Interaktion (28, 29, 31) einer Bedienperson mit der projizierten Bedienoberfläche (7, 11, 12), wobei an dem Darstellungselement (5, 8, 9) eine von der Erfassungseinrichtung separate Sensorik zum unabhängigen Erfassen der Interaktion (28, 29, 31) angeordnet ist,
**dadurch gekennzeichnet, dass**
- das Darstellungselement (5, 8, 9) eine maschinenlesbare Kennzeichnung (13, 14, 15) aufweist, welche ein Layout und/oder eine Funktionsauswahl der Bedienoberfläche (7, 11, 12) kodiert, wobei
- die Bedienvorrichtung (3) dazu eingerichtet ist, die Kennzeichnung (13, 14, 15) zu lesen und die projizierte Bedienoberfläche (7, 11, 12) in Abhängigkeit von dem kodierten Layout und/oder von der kodierten Funktionsauswahl anzupassen.

2. Bedienvorrichtung (3) nach Anspruch 1,
**dadurch gekennzeichnet, dass**
das Darstellungselement (5, 8, 9) ein Oberteil und Unterteil umfasst, wobei das Unterteil die Sensorik umfasst und das Oberteil beschädigungsfrei und reversibel von dem Unterteil lösbar an diesem gehalten ist.

3. Bedienvorrichtung (3) nach Anspruch 2,
**dadurch gekennzeichnet, dass**
das Oberteil aus einem beschädigungsfrei dampfsterilisierbaren und/oder autoklavierbaren Werkstoff, insbesondere einem Kunststoff, einem keramischen oder metallischen Werkstoff, gebildet ist.

4. Bedienvorrichtung (3) nach einem der vorhergehenden Ansprüche,
**dadurch gekennzeichnet, dass**
die Sensorik dazu eingerichtet ist, die Interaktion (28, 29, 31) mittels einer Auswertung einer Berührung des Darstellungselements (5, 8, 9) durch die Bedienperson zu erfassen.

5. Bedienvorrichtung (3) nach einem der vorhergehenden Ansprüche,
**dadurch gekennzeichnet, dass**
die Bedienoberfläche (7, 11, 12) in mehrere Sektoren unterteilt ist und
die Sensorik mehrere verteilt angeordnete Sensoren sowie eine Auswerteeinrichtung umfasst, welche dazu eingerichtet ist, mittels einer Auswertung von Sensordaten der einzelnen Sensoren zu bestimmen, in welchem Sektor die Interaktion (28, 29, 31) erfolgt.

6. Bedienvorrichtung (3) nach einem der vorhergehenden Ansprüche,
**dadurch gekennzeichnet, dass**
die Sensorik mit einer Aktuatorik kombiniert ist, welche dazu eingerichtet ist, als Reaktion auf eine erfasste Interaktion (28, 29, 31) ein haptisches Signal an dem Darstellungselement (5, 8, 9) zu erzeugen.

7. Bedienvorrichtung (3) nach einem der vorhergehenden Ansprüche,
**dadurch gekennzeichnet, dass**
die Projektionseinrichtung (4) ein Head-Mounted Display, insbesondere eine Augmented-Reality-Brille (4), umfasst.

8. Bedienvorrichtung (3) nach einem der vorhergehenden Ansprüche,
**dadurch gekennzeichnet, dass**
- die, insbesondere einen QR-Code (13, 14, 15) umfassende, maschinenlesbare Kennzeichnung (13, 14, 15) einen Typ des Darstellungselements (5, 8, 9) angibt, und
- die Projektionseinrichtung (4) dazu eingerichtet ist, die zu projizierende Bedienoberfläche (7, 11, 12) in Abhängigkeit von dem Typ des Darstellungselements (5, 8, 9) aus einer Vielzahl von vorgegebenen unterschiedlichen Bedienoberflächen (7, 11, 12) auszuwählen.

9. Bedienverfahren (3) zum Bedienen eines medizinischen Gerätes (1) mittels einer Bedienvorrichtung (3), bei dem
- eine Bedienoberfläche (7, 11, 12) für das medizinische Gerät (1) reell oder virtuell auf ein Darstellungselement (5, 8, 9) der Bedienvorrichtung (3) projiziert wird,
- berührungslos eine Interaktion (28, 29, 31) der Bedienperson mit der projizierten Bedienoberfläche (7, 11, 12) erfasst wird,
- die Interaktion (28, 29, 31) unabhängig von dem berührungslosen Erfassen der Interaktion (28, 29, 31) detektiert wird, und
- von der Bedienvorrichtung (3) in Abhängigkeit von der erfassten und detektierten Interaktion (28, 29, 31) ein Steuersignal für das medizinische Gerät (1) generiert und an das medizinische Gerät (1) übermittelt wird,
**dadurch gekennzeichnet, dass**
- von der Bedienvorrichtung (3) eine an dem Darstellungselement (5, 8, 9) angeordnete maschinenlesbare Kennzeichnung (13, 14, 15) gelesen wird, welche ein Layout und/oder eine Funktionsauswahl der Bedienoberfläche (7, 11, 12) kodiert, und
- die projizierte Bedienoberfläche (7, 11, 12) in Abhängigkeit von dem kodierten Layout und/oder von der kodierten Funktionsauswahl angepasst wird.

10. Bedienverfahren nach Anspruch 9,
**dadurch gekennzeichnet, dass**
- mittels der Bedienvorrichtung (3) eine Plausibilitätsprüfung jeweiliger die Interaktion (28, 29, 31) charakterisierender Ergebnisse des Erfassens und des Detektierens durchgeführt wird,
- das Steuersignal für das medizinische Gerät (1) nur dann an dieses übermittelt wird, wenn die Plausibilitätsprüfung ergibt, dass die beiden Ergebnisse zueinander plausibel sind.

## Claims

1. Operating apparatus (3) for a medical device (1), with a projection facility (4), which is configured for real and/or virtual projection of an operator surface (7, 11, 12) onto a display element (5, 8, 9), and with a capture facility for non-contact capturing of an interaction (28, 29, 31) of an operator with the projected operator surface (7, 11, 12), wherein
a sensor system for independent capturing of the interaction (28, 29, 31) separate from the capture facility is arranged on the display element (5, 8, 9),
**characterised in that**
- the display element (5, 8, 9) has a machine-readable code (13, 14, 15), which encodes a layout and/or a function selection of the operator surface (7, 11, 12), wherein
- the operating apparatus (3) is configured to read the code (13, 14, 15) and to adapt the projected operator surface (7, 11, 12) as a function of the encoded layout and/or the encoded function selection.

2. Operating apparatus (3) according to claim 1,
**characterised in that**
the display element (5, 8, 9) comprises an upper part and a lower part, wherein the lower part comprises the sensor system and the upper part is held on the lower part so as to be able to be released without damage and reversibly from said part.

3. Operating apparatus (3) according to claim 2,
**characterised in that**
the upper part is formed from a damage-free steam-sterilisable and/or autoclavable material, in particular a plastic, a ceramic or metallic material.

4. Operating apparatus (3) according to one of the preceding claims,
**characterised in that**
the sensor system is configured to capture the interaction (28, 29, 31) by means of evaluating a touch on the display element (5, 8, 9) by the operator.

5. Operating apparatus (3) according to one of the preceding claims,
**characterised in that**
the operator surface (7, 11, 12) is subdivided into a number of sectors and
the sensor system comprises a number of sensors arranged in a distributed manner as well as an evaluation facility, which is configured to determine the sector in which the interaction (28, 29, 31) is occurring by means of an evaluation of sensor data of the individual sensors.

6. Operating apparatus (3) according to one of the preceding claims,
**characterised in that**
the sensor system is combined with an actuator system, which is configured, as a reaction to a captured interaction (28, 29, 31), to create a haptic signal on the display element (5, 8, 9).

7. Operating apparatus (3) according to one of the preceding claims,
**characterised in that**
the projection facility (4) comprises a head-mounted display, in particular an augmented-reality headset (4).

8. Operating apparatus (3) according to one of the preceding claims,
**characterised in that**
- the machine-readable code (13, 14, 15), in particular comprising a QR code (13, 14, 15), specifies a type of the display element (5, 8, 9), and
- the projection facility (4) is configured to select the operator surface (7, 11, 12) to be projected as a function of the type of the display element (5, 8, 9) from a plurality of predetermined different operator surfaces (7, 11, 12).

9. Operating method (3) for operating a medical device (1) by means of an operating apparatus (3), in which
- an operator surface (7, 11, 12) for the medical device (1) is projected as a real or virtual surface onto a display element (5, 8, 9) of the operating apparatus (3),
- an interaction (28, 29, 31) of the operator with the projected operator surface (7, 11, 12) is captured in a non-contact manner,
- the interaction (28, 29, 31) is detected independently of the non-contact capturing of the interaction (28, 29, 31), and
- a control signal for the medical device (1) is generated and transferred to the medical device (1) by the operating apparatus (3) as a function of the captured and detected interaction (28, 29, 31),
**characterised in that**
- a machine-readable code (13, 14, 15), which is arranged on the display element (5, 8, 9) and encodes a layout and/or a function selection of the operator surface (7, 11, 12), is read by the operating device (3), and
- the projected operator surface (7, 11, 12) is adapted as a function of the encoded layout and/or the encoded function selection.

10. Operating method according to claim 9,
**characterised in that**
- a plausibility check of respective results of the capturing and of the detection characterising the interaction (28, 29, 31) is carried out by means of the operating apparatus (3),
- the control signal for the medical device (1) is only transferred to said device when the result of the plausibility check is that the two results are plausible in relation to one another.

## Revendications

1. Système (3) de commande d'un appareil (1) médical, comprenant un dispositif (4) de projection, qui est conçu pour la projection réelle et/ou virtuelle d'une surface (7, 11, 12) de commande sur un élément (5, 8, 9) de représentation, et comprenant un dispositif de détection pour la détection sans contact d'une interaction (28, 29, 31) d'un opérateur avec la surface (7, 11, 12) de commande projetée, dans lequel sur l'élément (5, 8, 9) de représentation est disposé, pour la détection indépendante de l'interaction (28, 29, 31), un ensemble capteur distinct du dispositif de détection,
**caractérisé en ce que**
- l'élément (5, 8, 9) de représentation a une caractéristique (13, 14, 15) déchiffrable par ordinateur, qui code un layout et/ou une sélection de fonction de la surface (7, 11, 12) de commande, dans lequel
- le système (3) de commande est conçu pour déchiffrer la caractérisation (13, 14, 15) et pour adapter la surface (7, 11, 12) de commande projetée en fonction du layout codé et/ou de la sélection de fonction codée.

2. Système (3) de commande suivant la revendication 1,
**caractérisé en ce que**
l'élément (5, 8, 9) de représentation comprend une partie supérieure et une partie inférieure, dans lequel la partie inférieure comprend l'ensemble capteur et la partie supérieure est maintenue, sans dommage et de façon réversible, par la partie inférieure de manière amovible sur celle-ci.

3. Système (3) de commande suivant la revendication 2,
**caractérisé en ce que**
la partie supérieure est en un matériau qui n'est pas dommageable, stérilisable à la vapeur et/ou autoclavable, notamment en une matière plastique, en un matériau céramique ou métallique.

4. Système (3) de commande suivant l'une des revendications précédentes,
**caractérisé en ce que**
l'ensemble capteur est conçu pour détecter l'interaction (28, 29, 31) au moyen d'une exploitation d'un toucher de l'élément (5, 8, 9) de représentation par l'opérateur.

5. Système (3) de commande suivant l'une des revendications précédentes,
**caractérisé en ce que**
la surface (7, 11, 12) de commande est subdivisée en plusieurs secteurs et
l'ensemble capteur comprend plusieurs capteurs répartis ainsi qu'un dispositif d'exploitation qui est conçu pour déterminer au moyen d'une exploitation de données des divers capteurs le secteur dans lequel a lieu l'interaction (28, 39, 31).

6. Système (3) de commande suivant l'une des revendications précédentes,
**caractérisé en ce que**
l'ensemble capteur est combiné à un ensemble actionneur, qui est conçu pour produire un élément haptique sur l'élément (5, 8, 9) de représentation en réaction à la détection d'une interaction (28, 29, 31).

7. Système (3) de commande suivant l'une des revendications précédentes,
**caractérisé en ce que**
le dispositif (4) de projection comprend un head-mounted display, notamment une lunette (4) à réalité augmentée.

8. Système (3) de commande suivant l'une des revendications précédentes,
**caractérisé en ce que**
- la caractérisation (13, 14, 15) pouvant être déchiffrée par ordinateur et comprenant notamment un code (13, 14, 15) QR indique un type de l'élément (5, 8, 9) de représentation, et
- le dispositif (4) de projection est conçu pour sélectionner la surface (7, 11, 12) de commande à projeter en fonction du type de l'élément (5, 8, 9) de représentation dans une pluralité de surfaces (7, 11, 12) de commande différentes données à l'avance.

9. Procédé (3) de commande pour la commande d'un appareil (1) médical au moyen d'un système (3) de commande, dans lequel
- on projette une surface (7, 11, 12) de commande de l'appareil (1) médical réellement ou virtuellement sur un élément (5, 8, 9) de représentation du système (3) de commande,
- on détecte sans contact une interaction (28, 29, 31) de l'opérateur avec la surface (7, 11, 12) de commande projetée,
- on détecte l'interaction (28, 29, 31) indépendamment du relevé sans contact de l'interaction (28, 29, 31), et
- on produit par le système (3) de commande en fonction de l'interaction (28, 29, 31) relevée et détectée un signal de commande de l'appareil (1) médical et on le transmet à l'appareil (1) médical,
**caractérisé en ce que**
- on lit par le système (3) de commande une caractérisation (13, 14, 15) déchiffrable par ordinateur et disposée sur l'élément (5, 8, 9) de représentation, qui code un layout et/ou une sélection de fonction de la surface (7, 11, 12) de commande, et
- on adapte la surface (7, 11, 12) de commande projetée en fonction du layout codé et/ou de la sélection de fonction codée.

10. Procédé de commande suivant la revendication 9,
**caractérisé en ce que**
- au moyen du système (3) de commande, on effectue un contrôle de vraisemblance respectivement des résultats, caractérisant l'interaction (28, 29, 31), du relevé et de la détection,
- on transmet le signal de commande de l'appareil (1) médical à celui-ci, seulement si le contrôle de vraisemblance indique que les deux résultats sont vraisemblables l'un par rapport à l'autre.
